# FASCICULE DE BREVET EUROPEEN

(11) **EP 3 927 346 B1**
(45) Date de publication et mention de la délivrance du brevet: **28.10.2026**
(21) Numéro de dépôt: 20712404.1
(22) Date de dépôt: 21.02.2020
(51) Int. Cl.: A61K 31/496, A61P 25/00, A61P 25/14

(54) **INHIBITEUR D'ACYL-THIOESTÉRASE APT1 POUR SON UTILISATION DANS LE TRAITEMENT ET/OU LA PRÉVENTION D'UNE MALADIE NEURODÉGÉNÉRATIVE OU D'UN TROUBLE NEURODÉGÉNÉRATIF**
APT1-ACYL-THIOESTERASE-HEMMER ZUR VERWENDUNG IN DER BEHANDLUNG UND/ODER PRÄVENTION NEURODEGENERATIVER ERKRANKUNGEN ODER NEUROGENERATIVER STÖRUNGEN
ACYL THIOESTERASE APT1 INHIBITOR FOR USE IN TREATING AND/OR PREVENTING A NEURODEGENERATIVE DISEASE OR A NEURODEGENERATIVE DISORDER

(30) Priorité: 21.02.2019 FR 1901773
(43) Date de publication de la demande: 29.12.2021
(73) Titulaire: Université Grenoble Alpes, 38400 Saint Martin d'Hères (FR); Institut National de la Santé et de la Recherche Médicale, 75013 Paris (FR); Centre Hospitalier Universitaire Grenoble Alpes, 38700 La Tronche (FR); Université Paris-Saclay, 91190 Gif sur Yvette (FR)
(72) Inventeur: SAUDOU, Frédéric, 38000 Grenoble (FR); VIRLOGEUX, Amandine, 69290 Craponne (FR)
(74) Mandataire: Cabinet Chaillot
(86) Numéro de dépôt international: PCT/IB2020/051476
(87) Numéro de publication internationale: WO 2020/170208

(56) Documents cités:
- WO-A2-2014/191727
- US-A1- 2018 147 250
- ALEXANDER ADIBEKIAN ET AL: "Confirming Target Engagement for Reversible Inhibitors in Vivo by Kinetically Tuned Activity-Based Probes", JOURNAL OF THE AMERICAN CHEMICAL SOCIETY, vol. 134, no. 25, 12 June 2012 (2012-06-12), pages 10345 - 10348, XP055638547, ISSN: 0002-7863, DOI: 10.1021/ja303400u
- ANAT YANAI ET AL: "Palmitoylation of huntingtin by HIP14is essential for its trafficking and function", NATURE NEUROSCIENCE, vol. 9, no. 6, 14 May 2006 (2006-05-14), US, pages 824 - 831, XP055638545, ISSN: 1097-6256, DOI: 10.1038/nn1702
- IGOR VUJIC ET AL: "Acyl protein thioesterase 1 and 2 (APT-1, APT-2) inhibitors palmostatin B, ML348 and ML349 have different effects on NRAS mutant melanoma cells", ONCOTARGET, vol. 7, no. 6, 9 February 2016 (2016-02-09), XP055638544, DOI: 10.18632/oncotarget.6907

## Description

### ARRIERE-PLAN DE L'INVENTION

La présente invention concerne le domaine du traitement et de la prévention des maladies neurodégénératives, et porte en particulier sur le traitement et la prévention de la maladie de Huntington.

Les maladies neurodégénératives ou troubles neurodégénératifs comprennent une large gamme de pathologies qui affectent principalement les neurones. Des exemples de maladies neurodégénératives et troubles neurodégénératifs qui sont liés à des défauts d'adressage des protéines et de leur trafic intracellulaire comprennent notamment, mais sans y être limité(e)s, la maladie de Huntington, la maladie de Parkinson, la Sclérose Latérale Amyotrophique ou maladie de Charcot (SLA), le syndrome de Rett, la maladie de Charcot-Marie-Tooth, la neuropathie axonale sensitivomotrice, le syndrome de Perry, la maladie d'Alzheimer précoce autosomique dominante (AD3), le syndrome de paralysie supranucléraire progressive (PSP), la paraplégie spastique autosomique.

La maladie de Huntington est une maladie neurologique d'origine génétique caractérisée par des déficits psychiatriques, moteurs et cognitifs apparaissant aux alentours de 30-40 ans chez l'Homme et par une dysfonction et une dégénérescence des neurones du cortex et du striatum. La maladie de Huntington touche en France environ 6000 personnes, et plus de 12000 personnes sont porteuses du gène muté, provisoirement indemnes de signes cliniques. C'est une maladie rare dont la prévalence, stable, est environ de 10 à 14 malades pour 100,000 au sein de la population caucasienne. Il n'existe pas à l'heure actuelle de traitement neuroprotecteur permettant de ralentir l'apparition ou la progression de la maladie. En particulier, aucune thérapie médicamenteuse non symptomatique n'est disponible dans le commerce. Des thérapies en développement actuellement se focalisent notamment sur des stratégies antisens ou de micro-ARN comme régulateurs traductionnels.

La maladie de Huntington a une transmission autosomique dominante et elle est à pénétrance complète. Il est admis que le porteur du gène muté développera la maladie au cours de sa vie. Le gène HD est situé chez l'Homme sur le chromosome 4p16.3. Il est composé de 67 exons et couvre 170 kb. Il code pour la protéine Huntingtine (HTT), laquelle remplit une fonction cruciale dans la régulation de la dynamique intracellulaire, notamment dans le transport axonal. Elle régule le trafic de vésicules et en particulier du BDNF (Brain Derived Neurotrophic Factor), un facteur neurotrophique essentiel.

Le gène muté contient une répétition du trinucléotide CAG (cytosine, adénine, guanine) instable qui code pour une expansion de polyglutamine (polyQ) dans la protéine Huntingtine HTT. Lorsque le gène code pour une protéine avec plus de 35 glutamines, il d déclenche une dysfonction puis une mort neuronale, notamment dans le cortex et le striatum. Les personnes porteuses de 39 répétitions à 60 sont certaines de développer la maladie de Huntington, dans ce cas la pénétrance est dite complète ; la pénétrance réduite est observée en présence de 36 à 39 répétitions (Rubinsztein et al., 1996). Les expansions de plus de 60 répétitions sont habituellement associées avec les formes juvéniles et adolescentes de la maladie de Huntington (Gencik et al., 2002). Il est admis que dans la maladie de Huntington, l'expansion polyQ conduit à un gain de nouvelles fonctions toxiques, mais également à une perte des fonctions neuroprotectrices de HTT sauvage. Avant même l'apparition des premiers symptômes, des dysfonctions existent au sein du réseau neuronal cortico-striatal.

Lorsque la Huntingtine est mutante, le transport du BDNF du cortex vers le striatum est réduit, conduisant à la dysfonction et à la mort des neurones du striatum et du cortex (Gauthier et al., 2004). Une des stratégies thérapeutiques est de restaurer ce transport afin de compenser le déficit d'apport neurotrophique et de ralentir la progression des symptômes de la maladie de Huntington.

La maladie de Huntington est généralement caractérisée par une triade de symptômes : moteurs, cognitifs et psychiatriques. Les troubles moteurs sont généralement divisés en deux phases : la première, la phase hyperkinétique, apparaît au cours de stades précoces de la maladie et est caractérisée par une importante chorée (Dorsey et al, 2013), la seconde, la phase hypokinétique, apparaît plus tardivement et est caractérisée par de la bradykinésie, de la dystonie, des troubles de l'équilibre et de la marche. Les troubles cognitifs se manifestent plusieurs années avant le début des symptômes moteurs. Une gamme de déficiences cognitives est observée dans de nombreux domaines comme la vitesse psychomotrice, la fonction exécutive, l'attention, la mémoire épisodique et de travail, l'apprentissage, les émotions et les odeurs (Paulsen, 2011 ; Papp et al., 2011). Enfin, une grande variété de symptômes neuropsychiatriques survient dans la maladie de Huntington, à savoir l'anxiété, l'irritabilité, l'apathie, des comportements obsessionnels compulsifs et la psychose.

On connaît à l'heure actuelle le lien entre la palmitoylation et la maladie de Huntington, tel que résumé dans la revue de Fiona B. Young et al., 2011. La palmitoylation est une modification importante pour l'adressage des protéines et leur trafic à l'intérieur des neurones. Elle correspond à l'ajout d'un acide gras, le palmitate, sur un résidu cystéine. Contrairement aux autres acylations telles que la myristoylation et l'isoprénylation, la palmitoylation est réversible. La palmitoylation augmente l'hydrophobicité des protéines et favorise donc les interactions avec la bicouche lipidique. Par conséquent, elle modifie l'adressage des protéines.

La palmitoylation est catalysée par une famille de protéines, les palmitoyl-acyl-transférases (PAT). Les gènes codant pour ces protéines sont hautement conservés parmi les espèces. L'une d'entre elles, HIP14, palmitoyle diverses protéines impliquées dans la plasticité synaptique, l'exocytose ou encore le développement.

Contrairement aux PAT, la découverte des enzymes qui catalysent la dépalmitoylation a été beaucoup moins rapide. A ce jour, six enzymes responsables de la dépalmitoylation ont été identifiées : PPT1 (protein-palmitoyl thioesterase), PPT2, APT1, APT2, APTL1 et les protéines ABHD17 (Tse et al., 2015 ; Tomatis et al., 2010 et Zeidman et al., 2009). APT1 et APT2 sont des protéines cytosoliques ayant des substrats communs. APT1 est exprimée dans de nombreux tissus chez la souris (Hirano et al., 2009). Elle a notamment un rôle dans la mémoire.

Dans le document WO 2014/191727 A2, il est d'ailleurs indiqué qu'un inhibiteur de la désacylation de la ghréline, par exemple un inhibiteur d'APT1, est utile dans le traitement ou la prophylaxie d'états physiologiques ou pathophysiologiques qui sont médiés par la ghréline acylée (AG) ou non-acylée (UAG) chez les mammifères, comme la maladie d'Alzheimer, la maladie de Parkinson, la démence, la sclérose en plaque, la maladie de Huntington, l'épilepsie, les troubles d'anxiété, la dépression, les troubles liés à l'alcool ou l'abus de drogues. Le document mentionne également qu'il est possible que l'inhibition d'autres enzymes qui provoquent la désacylation de la ghréline, telle que APT2 entre autres, peut être efficace dans ce contexte. En particulier, des composés qui inhibent à la fois APT1 et APT2 peuvent être utilisés. Les inhibiteurs divulgés dans ce document sont par exemple la Palmostatine B, la Palmostatine M et la tétrahydrolipastine, ainsi que des composés à base de bore comme les acides boroniques et les acides boriniques et leurs dérivés.

Des travaux suggèrent une altération de la palmitoylation lorsque la Huntingtine (HTT) est mutante et que la réexpression d'une enzyme appelée HIP14 promouvant la palmitoylation pourrait inhiber la mort des cellules. Cependant, la surexpression de HIP14 a des effets toxiques et il n'existe pas de molécules stimulant cette enzyme. La surexpression de HIP14 dans des neurones corticaux augmente la palmitoylation de HTT et relocalise HTT à l'appareil de Golgi, suggérant que la localisation de HTT au Golgi est dépendante de sa palmitoylation (Yanaï et al., 2006).

Les présents inventeurs ont utilisé une stratégie inverse consistant à inhiber les enzymes responsables de la dépalmitoylation, les acyl-thioestérases, pour lesquelles il existe des molécules sélectives. Jusqu'ici les travaux publiés concernent le rétablissement de l'activité de HIP14 dans le traitement de la maladie de Huntington sans décrire le processus de dépalmitoylation comme nouvel axe thérapeutique.

Comme indiqué ci-dessus, le lien entre la palmitoylation et la maladie de Huntington est bien connu (Yanaï et al., 2006), tout comme les enzymes impliquées dans ce processus : HIP14 et HIP14L pour la palmitoylation des protéines, y compris la palmitoylation de la protéine Huntingtine HTT. Les enzymes régulant la réaction inverse, la dépalmitoylation, sont les thioestérases, dont font partie APT1 et APT2 (Young et al., 2012). D'autres publications décrivent par ailleurs des molécules inhibitrices des thioestérases dont ML-348 (Adibekian et al., 2012 et Vujic et al., 2015).

Si l'on connaît, notamment de la publication de Adibekian et al., 2012, des molécules inhibitrices de thioestérase et en particulier ML-348, le potentiel thérapeutique de ce dernier n'est pas évoqué. Par ailleurs, il ressort des travaux de Vujic et al., 2015, que les inhibiteurs ML-348 (spécifique de APT1) et ML-349 (spécifique de APT2) n'ont pas d'effet biologiquement significatif sur des cellules de mélanomes, par opposition à la Palmostatine B. Ces études restent muettes sur le potentiel en recherche thérapeutique sur les maladies neurodégénératives, notamment sur la maladie de Huntington.

Il n'existe pas actuellement de molécule permettant le rétablissement du trafic intracellulaire qui est altéré dans une maladie neurodégénérative telle que la maladie de Huntington. La présente invention a pour objet de mettre en évidence le rôle important de l'inhibiteur de l'acyl-thioestérase APT1, ML-348, dans le traitement et la prévention de la maladie de Huntington. Ses effets, tant sur les déficits cognitifs que sur les déficits moteurs, montrent le nouveau potentiel thérapeutique et prophylaxique de ce composé sur la maladie de Huntington. Cet inhibiteur présente un intérêt majeur car il n'existe pour le moment aucune thérapie médicamenteuse neuroprotectrice pour soigner la maladie de Huntington.

### BREVE DESCRIPTION DE L'INVENTION

Afin de pouvoir cribler des molécules en rapport à la dynamique intracellulaire globale dans les neurones, les inventeurs ont adapté un système appelé RUSH (Retention Using Selective Hooks) qui permet de suivre le trafic vésiculaire depuis le réticulum endoplasmique (RE) jusqu'à la membrane plasmique (Boncompain et al., 2012), ceci dans des cellules sauvages et mutantes pour la maladie de Huntington. Ils ont observé que la palmostatine B, un inhibiteur non spécifique des acyl-thiolestérases était capable de restaurer le trafic intracellulaire de manière comparable à des cellules sauvages. Ils ont alors testé d'autres molécules inhibitrices sélectives pour différentes acyl-thioestérases (APT1 et APT2) présentes dans les neurones.

Ainsi, ils ont pu montrer que seule la molécule ML-348 spécifique de APT1 était efficace. Grâce à l'approche microfluidique *in vitro* qui consiste à fabriquer, dans un matériau biocompatible et transparent, des chambres de culture et des canaux à l'échelle des cellules, ils ont reconstitué le circuit cortico-striatal normal et dysfonctionnel dans la maladie de Huntington. Ce système étant compatible avec la microscopie rapide à haute résolution, il permet d'étudier des mécanismes de trafic intracellulaire dans un réseau neuronal mature et fonctionnel.

Les résultats détaillés ci-après montrent que :
- l'inhibition d'APT1 par ML-348 restaure le transport du BDNF du cortex vers le striatum, suggérant que ML-348 est une molécule d'intérêt thérapeutique pour la maladie de Huntington ;
- ML-348 traverse la barrière hémato-encéphalique : chez des souris Htt^{CAG140/+} modèles de la maladie de Huntington et âgées de 5 mois, traitées par l'inhibiteur ML-348, il a pu être montré que ce traitement restaure les altérations comportementales liées à la maladie, aussi bien au niveau de la motricité qu'au niveau de l'anxiété.

Les inventeurs, après identification de l'inhibiteur ML-348 comme molécule restaurant les cinétiques de la voie de sécrétion, ont évalué son effet sur le réseau cortico-striatal *in vitro.* ML-348 est capable d'améliorer les déficits de transport axonal du BDNF, les défauts de densité synaptique et de signalisation striatale dans la maladie de Huntington. Le traitement de neurones sains ne montre pas de défauts du réseau cortico-striatal, hormis au niveau du nombre de vésicules de BDNF transportées le long de l'axone. La potentielle multiplicité des substrats d'APT1 ne permet pas de conclure que la restauration du fonctionnement du réseau sur puce microfluidique soit due uniquement au rétablissement des cinétiques de transport dans les voies de sécrétion. Cependant, étant donné le rôle du transport axonal et des afférences corticales dans le fonctionnement du réseau, il semble que le rétablissement du trafic soit essentiel au mécanisme de restauration.

La présente invention a donc pour objet un inhibiteur de l'acyl-thioestérase APT1, ML-348, de formule A : 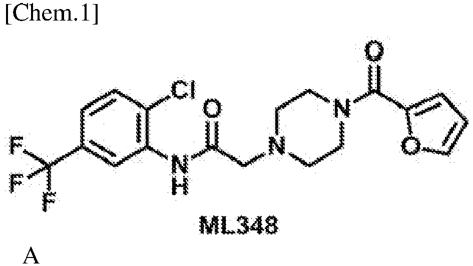 pour son utilisation dans le traitement et/ou la prévention d'une maladie neurodégénérative ou d'un trouble neurodégénératif.

De telles maladies neurodégénératives ou troubles neurodégénératifs comprennent, mais sans y être limités, la maladie de Huntington, la maladie de Parkinson, la Sclérose Latérale Amyotrophique ou maladie de Charcot (SLA), le syndrome de Rett, la maladie de Charcot-Marie-Tooth, la neuropathie axonale sensitivomotrice, le syndrome de Perry, la maladie d'Alzheimer précoce autosomique dominante (AD3), le syndrome de paralysie supranucléraire progressive (PSP), la paraplégie spastique autosomique.

De manière préférée, la maladie neurodégénérative ou le trouble neurodégénératif est la maladie de Huntington.

La maladie neurodégénérative ou le trouble neurodégénératif peut également être le syndrome de Rett. En effet, cette maladie génétique est liée à la mutation d'un gène porté par le chromosome X, le gène MECP2. La protéine codée par ce gène a une fonction importante dans le développement et le fonctionnement harmonieux des neurones et parmi les voies physiologiques altérées, on trouve notamment celle du BDNF. Comme indiqué plus haut, la Huntingtine joue un rôle crucial dans la régulation du trafic de ce facteur neurotrophique essentiel.

En outre, l'inhibiteur de l'acyl-thioesthérase APT1 de la présente invention est capable de se lier à l'acyl-thioesthérase APT1.

Ainsi, la liaison de ML-348 permet d'empêcher le substrat de se fixer sur le site actif de l'enzyme acyl-thioesthérase APT1 et/ou l'enzyme APT1 de catalyser sa réaction de dépalmitoylation.

L'invention concerne également une composition pharmaceutique comprenant :
- au moins l'inhibiteur de l'acyl-thioestérase APT1 de la présente invention ; et
- au moins un excipient pharmaceutiquement acceptable pour son utilisation dans le traitement et/ou la prévention d'une maladie neurodégénérative ou d'un trouble neurodégénératif.

L'expression "excipient pharmaceutiquement acceptable" se réfère à un matériel non toxique qui est compatible avec un système biologique tel qu'une cellule, une culture cellulaire, un tissu ou un organisme. Cet excipient pharmaceutiquement acceptable ne produit pas de réaction indésirable, allergique ou autre lorsqu'il est administré à un animal, en particulier un être humain. Les caractéristiques de l'excipient dépendront du mode d'administration utilisé.

Ceci comprend n'importe quel solvant, diluant, milieu de dispersion, agglutinant, liant, lubrifiant, délitant, revêtement, agent antibactérien et antifongique, agent isotonique et agent retardateur d'absorption, et adjuvants similaires. Un excipient pharmaceutiquement acceptable se réfère à une charge solide, semi-solide ou liquide nontoxique, un diluant, une matière d'encapsulation ou une formulation accessoire de tout type. Pour l'administration humaine, les préparations devront satisfaire les exigences de stérilité, pyrogénicité, de sûreté générale et de pureté telles que requises par les Bonnes pratiques de fabrication des substances actives à usage humain et vétérinaire.

Selon l'invention, l'inhibiteur de l'acyl-thioestérase APT1 ou la composition est destiné(e) à être administré(e) au sujet selon une quantité thérapeutiquement efficace.

L'expression « quantité thérapeutiquement efficace » signifie le taux ou la quantité de composé nécessaire et suffisante pour ralentir ou stopper la progression, l'aggravation ou la détérioration d'un ou plusieurs symptômes de la maladie ou du trouble, en particulier la maladie neurodégénérative ou le trouble neurodégénératif, plus particulièrement la maladie de Huntington; soulager les symptômes de la maladie ou du trouble, en particulier la maladie neurodégénérative ou le trouble neurodégénératif, plus particulièrement la maladie de Huntington.

La « quantité thérapeutiquement efficace » dépend du sujet, du stade de la maladie à traiter et du mode d'administration, et peut être déterminée par des opérations de routine par l'homme du métier.

Une « quantité thérapeutiquement efficace » correspond à une quantité suffisante pour diminuer les symptômes de la maladie et l'évolution de celle-ci. Cette quantité peut varier avec l'âge, le sexe du sujet et le stade de la maladie et sera déterminée par l'homme du métier.

Avantageusement, une quantité thérapeutiquement efficace peut varier entre 0,01 et 100 mg/kg de masse corporelle, de préférence entre 0,1 et 20 mg/kg, et de façon davantage préférée entre 0,1 et 2 mg/kg, en une ou plusieurs administrations quotidiennes, pendant un ou plusieurs jours.

En outre, la dose thérapeutiquement efficace spécifique pour n'importe quel patient dépendra d'une diversité de facteurs comprenant le trouble traité et la gravité du trouble ; l'activité du composé spécifique utilisé ; la composition spécifique utilisée, l'âge, la masse corporelle, l'état de santé général, le sexe et le régime du patient ; la durée d'administration, la voie d'administration, et le taux d'excrétion du composé spécifique utilisé ; la durée du traitement ; les médicaments utilisés en combinaison ou simultanément avec le composé spécifique utilisé ; et les facteurs similaires bien connus dans la technique médicale. Par exemple, il est bien dans les compétences de l'homme du métier de commencer des doses du composé à des taux inférieurs à ceux requis pour obtenir l'effet thérapeutique désiré et d'augmenter progressivement le dosage jusqu'à ce que l'effet désiré soit obtenu. Cependant, le dosage quotidien des produits peut être modifié sur une large gamme allant d'environ 10 à environ 10000 mg par adulte par jour, de préférence de 100 à environ 5000, de façon davantage préférée d'environ 200 à environ 2000 mg par adulte par jour. De préférence, les compositions contiennent 10, 50, 100, 250, 500, 1000 et 2000 mg du principe actif pour l'ajustement symptomatique du dosage au patient à traiter. Un médicament typiquement contient d'environ 10 à environ 10000 mg du principe actif, de préférence 100 à environ 5000, de façon davantage préférée d'environ 200 à environ 2000 mg du principe actif. Une quantité efficace du médicament est d'ordinaire fournie à un dosage allant de 0,1 mg/kg à environ 100 mg/kg de masse corporelle par jour, de préférence d'environ 1 mg/kg à 40 mg/kg de masse corporelle par jour, de façon davantage préférée d'environ 2 mg/kg à 20 mg/kg de masse corporelle par jour.

Dans les compositions pharmaceutiques de la présente invention, le principe actif, seul ou en combinaison avec un autre principe actif, peut être administré sous une forme d'administration unitaire, sous la forme d'un mélange avec des supports pharmaceutiques classiques, à des animaux et des êtres humains. Les formes d'administration unitaires appropriées comprennent les formes adaptées à la voie orale telles que les comprimés, les gélules, les poudres, les granules et les suspensions ou solutions pour voie orale, les formes d'administration sublinguale et buccale, les aérosols, les implants, les formes d'administration sous-cutanée, transdermique, topique, intrapéritonéale, intramusculaire, intraveineuse, sous-dermique, transdermique, intrathécale et intranasale et les formes d'administration rectale.

Dans un mode de réalisation, la composition pharmaceutique contient des véhicules qui sont pharmaceutiquement acceptables pour une formulation adaptée pour l'administration par voie orale.

Des exemples de formes adaptées pour l'administration par voie orale comprennent, mais sans y être limitées, des comprimés, des comprimés à orodispersion, des comprimés effervescents, des poudres, des granules, des pilules (comprenant des pilules édulcorées), des dragées, des gélules (comprenant des gélules de gélatine molle), des sirops, des liquides, des gels ou d'autres solutions, des suspensions, des bouillies, des formes liposomales et similaires.

Dans un mode de réalisation, la composition pharmaceutique contient des véhicules qui sont pharmaceutiquement acceptables pour une formulation apte à être injectée.

Des exemples de formes adaptées à l'injection comprennent, mais sans y être limitées, des solutions, telles que, par exemple, des solutions aqueuses stériles, des dispersion, des émulsions, des suspensions, des formes solides appropriées pour l'utilisation pour préparer des solutions ou suspensions par l'ajout d'un liquide avant l'utilisation, par exemple, une poudre, des formes liposomales ou similaires.

Le mode d'administration peut être par injection ou par perfusion graduelle. L'injection peut être intraveineuse, intra-péritonéale, intramusculaire, sous-cutanée ou transdermale.

Les préparations pour une administration parentérale peuvent inclure des solutions aqueuses ou non-aqueuses stériles, des suspension ou des émulsions. Des exemples de solvants non-aqueux sont le propylène glycol, le polyéthylène glycol, des huiles végétales ou des esters organiques injectables tels que l'éthyl oléate. Des véhicules aqueux comprennent l'eau, des solutions alcool/eau, des émulsions ou des suspensions.

Dans un mode de réalisation particulier, l'inhibiteur de l'acyl-thioestérase APT1, ML-348, ou la composition pharmaceutique est destiné(e) une utilisation dans le traitement et/ou la prévention d'une maladie ou d'un trouble lié(e) à un trafic intracellulaire altéré entre le réticulum endoplasmique et l'appareil de Golgi et entre celui-ci et la membrane plasmique.

Pour mieux illustrer l'objet de la présente invention, on va maintenant en décrire ci-dessous, à titre illustratif et non limitatif, les exemples ci-après en liaison avec les dessins annexés.

Sur ces dessins :
[Fig.1A] est une représentation schématique du système RUSH KDEL-GPI-mCherry.
[Fig.1B] est un graphique représentant la quantification de la fluorescence du Golgi à chaque instant dans les cellules striatales StHdhQ7/Q7 (ronds) et StHdhQ111/Q111 (carrés). Les panels sous les courbes représentent le trafic de GPI à l'intérieur de l'appareil de Golgi.
[Fig.1C] représente la cinétique des cellules exprimant le plasmide GPI-KDEL-mCherry à partir de l'ajout de biotine à t=0 jusqu'à 90 minutes après cet ajout de biotine (barre d'échelle=10 µm).
[Fig.2A] est une représentation schématique de la réaction de palmitoylation/dépalmitoylation catalysée par les palmitoyl-acyl-transférases (PAT) et les acyl-thioestérases (dePAT). Les principales dePAT et leurs inhibiteurs sont indiqués dans le panel de droite.
[Fig.2B] est un graphique représentant la quantification de fluorescence dans le Golgi en fonction du temps après l'ajout de biotine sur des cellules StHdhQ7/Q7 (ronds), StHdhQ111/Q111 (carrés) et StHdhQ111/Q111 traitées 1 heure par la Palmostatine B à 10µM (triangles).
[Fig.2C] est un graphique représentant la quantification de fluorescence dans le Golgi en fonction du temps après l'ajout de biotine sur des cellules StHdhQ7/Q7 (ronds), StHdhQ111/Q111 (carrés) et StHdhQ111/Q111 traitées 1 heure par ML-348 à 10µM (triangles).
[Fig.2D] est un graphique représentant la quantification de fluorescence dans le Golgi en fonction du temps après l'ajout de biotine sur des cellules StHdhQ7/Q7 (ronds), StHdhQ111/Q111 (carrés) et StHdhQ111/Q111 traitées 1 heure par ML-349 à 10µM (triangles).
[Fig.2E] est une représentation schématique de la réaction de palmitoylation/dépalmitoylation catalysée par les palmitoyl-acyl-transférases (PAT) et les acyl-thioestérases (dePAT). Le silençage génique des acyl-thioestérases est schématisé dans le panel de droite.
[Fig.2F] est un graphique représentant la quantification de fluorescence dans le Golgi en fonction du temps après l'ajout de biotine sur des cellules StHdhQ7/Q7 avec si-CTRL (ronds), StHdhQ111/Q111 avec si-CTRL (carrés) et StHdhQ111/Q111 avec si-APT1 (triangles).
[Fig.2G] est un graphique représentant la quantification de fluorescence dans le Golgi en fonction du temps après l'ajout de biotine sur des cellules StHdhQ7/Q7 avec si-CTRL (ronds), StHdhQ111/Q111 avec si-CTRL (carrés) et StHdhQ111/Q111 avec si-APT2 (triangles).
[Fig.3A] est une représentation schématique des dispositifs microfluidiques utilisés pour reconstruire le réseau cortico-striatal in vitro et les conditions de traitement.
[Fig.3B] est une représentation schématique de la zone d'enregistrement vidéo pour le trafic de BDNF-mCherry.
[Fig.3C] représente des kymographes montrant le trafic axonal de BDNF-mCherry obtenu à partir de cellules de type sauvage et de cellules HdhCAG/+ traitées ou non par ML-348.
[Fig.3D] est un graphique représentant les vélocités antérogrades et rétrograde du transport de BDNF-mCherry.
[Fig.3E] est un graphique représentant le nombre de vésicules antérogrades et rétrogrades en déplacement le long de 100µm d'axone.
[Fig.3F] est un graphique représentant la vitesse d'échoulement
[Fig.3G] est un graphique représentant le flux net linéaire.
[Fig.3H] est une représentation schématique du dispositif microfluidique et de la zone d'enregistrement pour DSD95 et la synaptophysine SYP.
[Fig.3I] est une représentation graphique du nombre de points de PSD95 et SYP adjacents le long de 100µm de neurite.
[Fig.3J] est une représentation schématique du dispositif microfluidique en situation de stimulation des neurones corticaux où un marquage phosphoERK (pERK) est réalisé à l'intérieur du compartiment postsynaptique.
[Fig.3K] est un graphique représentant la quantification des cellules immunopositives pour pERK.
[Fig.3L] est une représentation graphique de la toxicité de ML-348 mise en évidence par un test MTT au sel de tétrazolium MTT (bromure de 3-(4,5-dimethylthiazol-2-yl)-2,5-diphenyl tetrazolium) : l'absorbance à 595-690 nm des cellules neuronales du cortex de souris en fonction de la concentration de ML-348 (de 100 nM à 100 µM) permet de quantifier le nombre de cellules vivantes et métaboliquement actives.
[Fig.3M] est une autre représentation graphique des résultats du test MTT sur des cellules neuronales de cortex de souris : témoin négatif (milieu neurobasal NB+B27), témoin positif (milieu neurobasal NB+B27 + 1% de DMSO), et cellules en présence de 1µM de ML-348 dans milieu neurobasal NB+B27.
[Fig.4A] est une représentation schématique de l'injection intrapéritonéale de ML-348 (2mg/kg) chez les souris, puis l'extraction des cerveaux à t= 0, 30, 60 et 180 minutes avant l'analyse de pharmacocinétique.
[Fig.4B] est une représentation graphique montrant la concentration plasmatique de ML-348 au cours du temps.
[Fig.4C] est une représentation graphique de la concentration de ML-348 dans le cerveau au cours du temps.
[Fig.4D] est une représentation schématique de l'immunoprécipitation d'une protéine palmitoylée par la méthode ABE (Acyl-Biotin-Exchange) dans laquelle un lysat de cerveau est soumis à ABE séquentiel pour biotinyler des protéines palmitoylées avant leur immunoprécipitation avec des billes revêtues de streptavidine.
[Fig.4E] représente la migration sur gel d'électrophorèse après ABE d'un lysat de cerveau provenant de souris injectées avec une solution témoin ou avec une solution de ML-348 à 0,3 mg/kg sur un gel sans marquage.
[Fig.4F] représente la migration sur gel d'électrophorèse après ABE d'un lysat de cerveau provenant de souris injectées avec une solution témoin ou avec une solution de ML-348 à 0,3 mg/kg en présence de streptavidine-HRP. La Figure 4F montre la palmitoylation des protéines cérébrales après injection de ML-348 en comparaison avec la situation témoin (CTRL).
[Fig.4G] est un gel d'électrophorèse montrant le taux constant d'APT1 avant et après l'injection de ML-348.
[Fig.5A] est une représentation schématique du programme expérimental (O/F : Open Field, HL : Horizontal Ladder, NSF : Novelty-Suppressed Feeding).
[Fig.5B] est un graphique représentant le nombre d'entrées dans la zone centrale d'un appareillage de test d'Open Field sur une période de 60 minutes.
[Fig.5C] est un graphique représentant la latence jusqu'à la prise de nourriture mesurée sur une période de 10 minutes après l'entrée dans la zone de test de NSF.
[Fig.5D] est un graphique représentant la locomotion spontanée des souris en tant que distance totale parcourue pendant 60 minutes dans le cadre du test d'Open Field.
[Fig.5E] est une représentation graphique du score d'erreurs moyen et de la durée moyenne de traversée de l'échelle pour le test d'Horizontal Ladder permettant de mettre en évidence la motricité.
[Fig.5F] est une représentation graphique de la vitesse moyenne atteinte par les souris sur un appareil rotarod à vitesse accélérée et du nombre de chutes sur un appareil rotarod à vitesse fixe.
[Fig.6A] est une représentation graphique de la densité synaptique mesurée sous la forme du nombre de points adjacents de PSD95 et Vglut-1 sur des neurones de souris de type sauvage vs HdhCAG/+.
[Fig.6B] présente les résultats du test ABE sur des neurones de souris de type sauvage vs HdhCAG/+ avec ou sans traitement par ML-348.
[Fig.7A] est une représentation schématique des dispositifs microfluidiques utilisés pour reconstruire le réseau cortico-striatal in vitro et les conditions de traitement.
[Fig.7B] représente des projections selon l'axe z et les kymographes associés montrant le trafic axonal de BDNF-mCherry obtenu à partir de neurones corticaux issus de cellules souches pluripotentes induites d'origine humaine, traitées par le DMSO (témoin) vs. ML-348.
[Fig.7C] est une représentation graphique des résultats d'analyse du transport de BDNF-mCherry, à savoir les vélocités antérograde et rétrograde du transport des vésicules de BDNF-mCherry, le nombre de vésicules antérogrades et rétrogrades en déplacement le long de 100µm d'axone et le flux net linéaire.

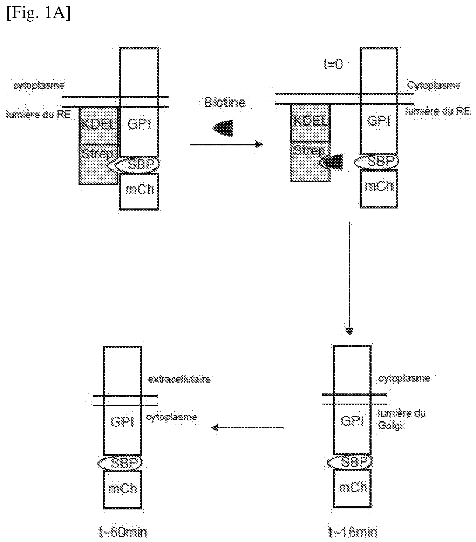

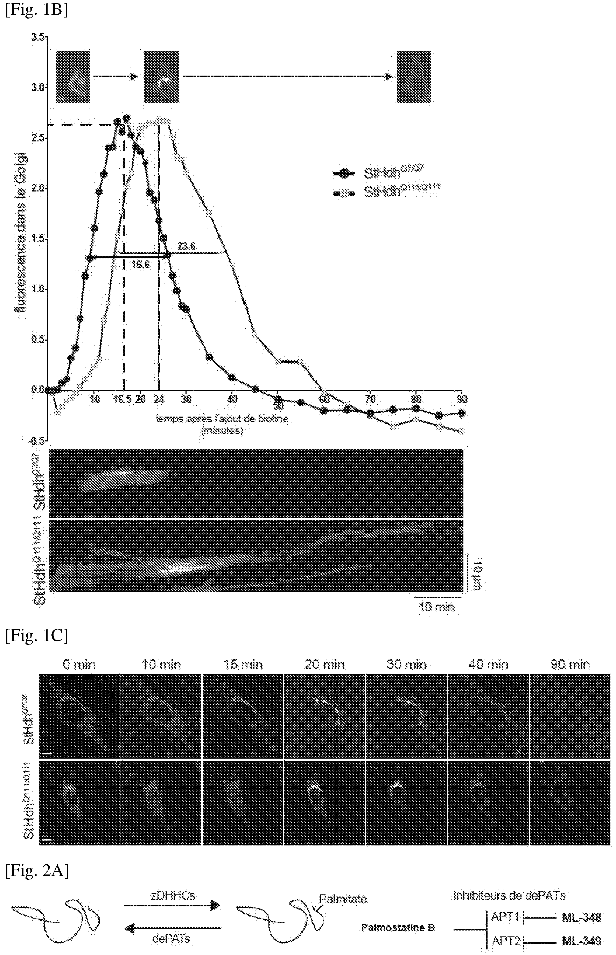

La Figure 1 montre que les dynamiques intracellulaires sont retardées dans la maladie de Huntington.

Si l'on se réfère à la Figure 1A, on peut voir que GPI (glycosylphosphatidyl inositol) est fusionné avec SBP (Streptavidin-Binding Peptide) et un marqueur fluorescent mCherry (mChr) pour constituer le « Rapporteur ». Le Rapporteur est retenu dans le réticulum endoplasmique à l'aide de la partie hameçon (KDEL-Streptavidine, en grisé). L'ajout de biotine à t=0 libère le Rapporteur et permet son trafic à travers la voie sécrétoire après avoir quitté le réticulum endoplasmique pour l'appareil de Golgi (t≈ 16 min) puis la membrane plasmique (t≈ 60 min).

Sur la Figure 1B, les inventeurs ont étudié le trafic du Rapporteur GPI-SBP-mCherry par imagerie en temps réel pendant 90 minutes dans les cellules St*Hdh*^{Q7/Q7} (ronds) et St *Hdh*^{Q111/Q111} (carrés). Les nombres sur l'axe X représentent le temps quand la fluorescence du Golgi est la plus forte pour chaque type de cellules. Les nombres indiqués sur la flèche horizontale indiquent la durée écoulée pour que la fluorescence traverse le Golgi pour chaque type de cellules. Il a ainsi été observé que le pic de fluorescence était à 16,6 minutes pour les cellules striatales de souris St*Hdh*^{Q7/Q7}, alors qu'il était retardé jusqu'à 23,6 minutes dans les cellules St*Hdh*^{*Q*111/Q111} reproduisant le phénotype de la maladie de Huntington. Du graphique et des images prises en microscopie à fluorescence ressort que le Rapporteur présente une rétention prolongée dans l'appareil du Golgi dans les cellules St*Hdh*^{Q111Ql111}*.*

Sur la Figure 1C, on peut également observer la cinétique d'apparition et de disparition de la fluorescence mCherry dans l'appareil de Golgi, avec un trafic réduit du réticulum endoplasmique vers le Golgi pour les cellules St*Hdh*^{Q111/Q111} ainsi que la rétention prolongée décrite plus haut, la détection de la fluorescence mCherry étant possible bien après 40 minutes après l'ajout de biotine.

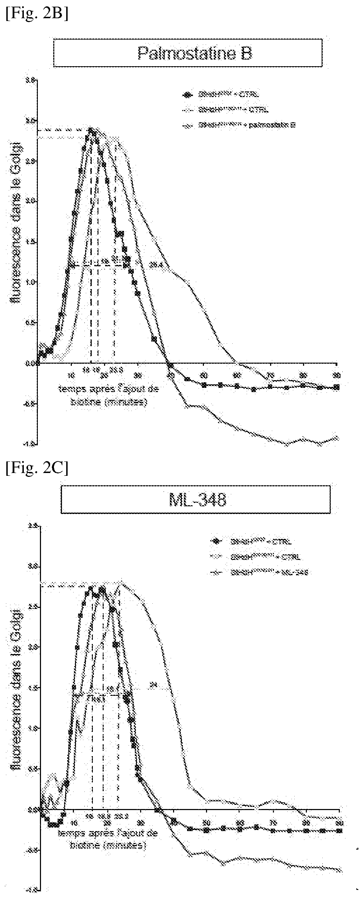

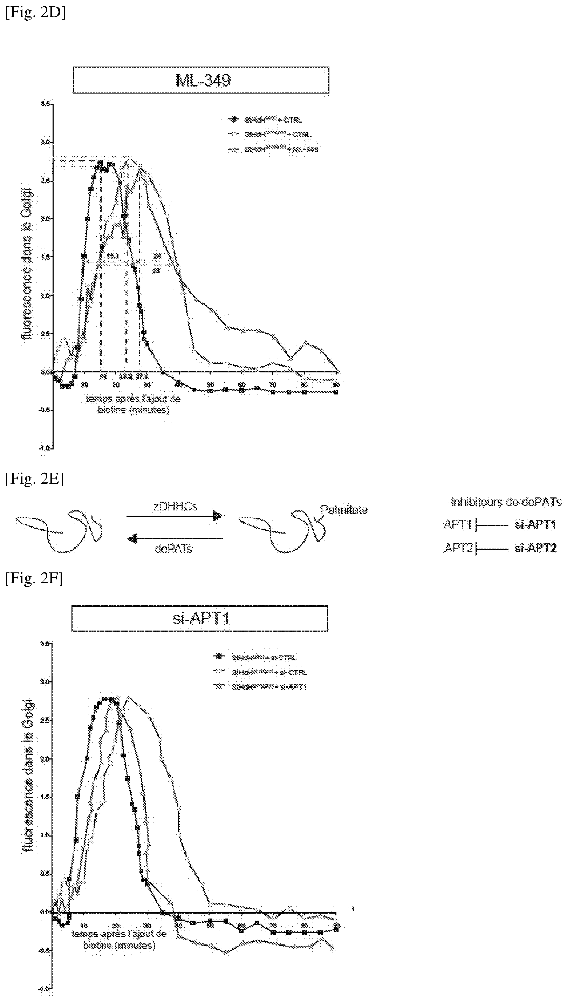

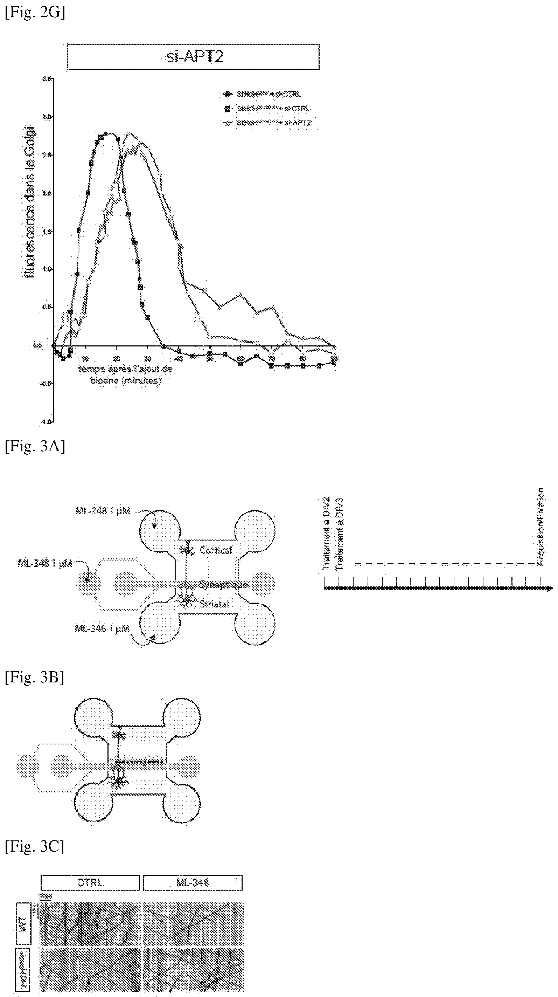

La Figure 2 montre que les inhibiteurs de l'acyl-thioestérase APT1 restaurent la dynamique intracellulaire dans la maladie de Huntington.

Si l'on se réfère à la Figure 2A, il existe des molécules inhibitrices des dépalmitoylases (acyl-thioestérases) APT1 et APT2 : la Palmostatine B, non spécifique, et ML-348, inhibiteur spécifique de APT1 et ML-349, inhibiteur spécifique de APT2.

Sur la Figure 2B, on peut constater que le traitement par la Palmostatine B à 10 µM pendant 1 heure sur les cellules St*Hdh*^{Q111/Q111} (triangles) restaure les cinétiques du réticulum endoplasmique vers l'appareil de Golgi : 18 minutes après l'ajout de biotine pour l'apparition du pic de fluorescence dans le Golgi vs 23,3 min sans traitement (carrés). Le traitement par la Palmostatine B diminue également la durée que prend la fluorescence pour quitter l'appareil de Golgi (21,3 min vs 25,4 minutes sans traitement).

Sur la Figure 2C, les inventeurs ont étudié les effets similaires de ML-348 sur les cinétiques : 18,9 min après l'ajout de biotine pour l'apparition du pic de fluorescence dans le Golgi vs 23,2 min sans traitement. Le traitement par ML-348 diminue aussi la durée de rétention dans le Golgi à une valeur identique à celle obtenue chez les cellules de type sauvage (15,1 minutes), en opposition à 24 minutes pour les cellules *StHdh* ^{Q111/Q111} non traitées.

En revanche, sur la Figure 2D, le traitement par ML-349 (inhibiteur spécifique de APT2) ne fait apparaître aucun effet sur les cinétiques du réticulum endoplasmique vers l'appareil de Golgi ni sur la durée de rétention dans le Golgi.

Ces résultats suggèrent que l'inhibition de APT1 plutôt que APT2 est capable de restaurer le trafic du réticulum endoplasmique vers la membrane plasmique qui est altéré dans les cellules affectées par la maladie de Huntington.

Les inventeurs ont ensuite procédé à démontrer que le traitement par les inhibiteurs spécifiques de APT1 et APT2 (Figure 2E) jouaient effectivement un rôle d'inhibition sur l'activité de ces enzymes. Le silençage génique par des ARNsi spécifiques (Figure 2F et 2G, triangles), si-APT1 et si-APT2, se traduit par la restauration du trafic du RE vers le Golgi et l'absence de rétention de la fluorescence mCherry dans l'appareil de Golgi dans le cas de si-APT1 (Figure 2F), et une arrivée plus tardive dans le Golgi et la rétention de la fluorescence à ce niveau dans le cas de si-APT2 (Figure 2G). Ces résultats soulignent que les inhibiteurs ML-348 et ML-349 utilisés dans l'expérience ont bien une action inhibitrice sur leurs enzymes spécifiques cibles, et que seule l'inhibition de APT1 a un effet sur les cellules affectées par la maladie de Huntington et est une cible d'intérêt pour la prévention et le traitement de la maladie.

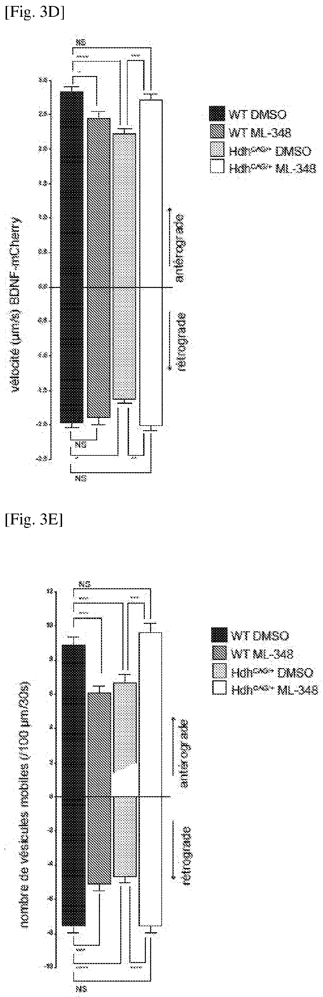

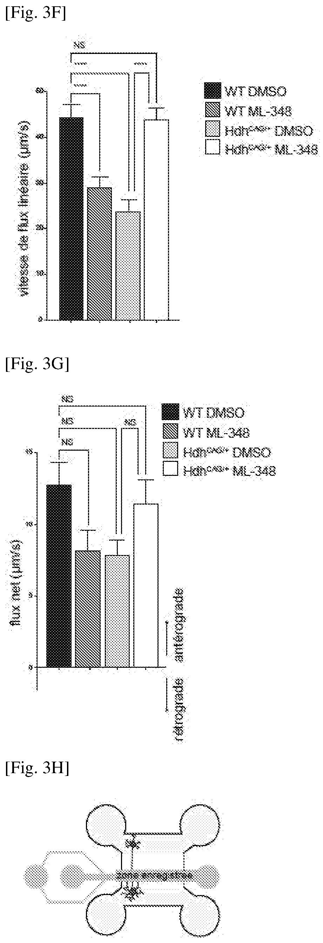

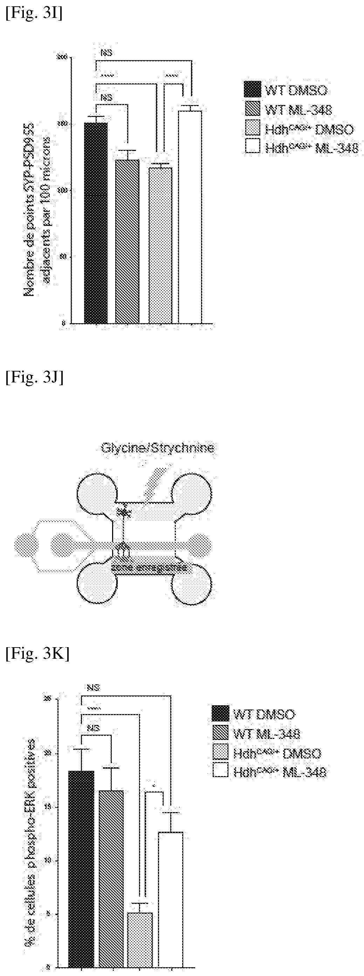

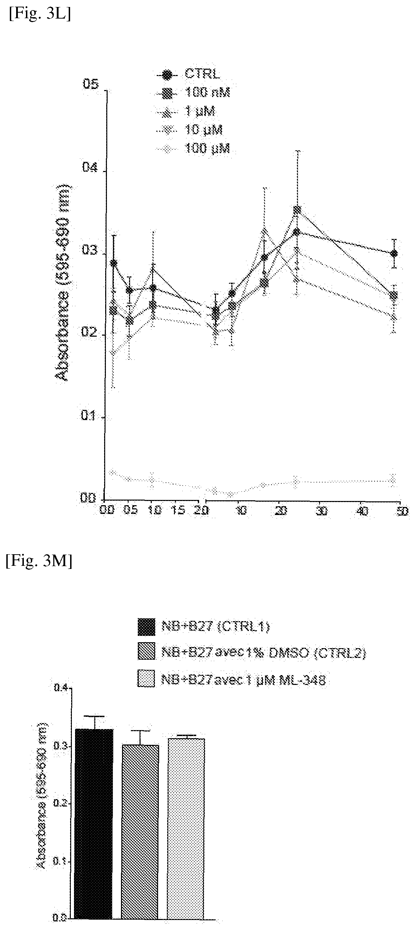

La Figure 3 montre l'effet d'un traitement chronique par ML-348 sur le réseau cortico-striatal.

Si l'on se réfère à la Figure 3A, les inventeurs ont reproduit le réseau cortico-striatal in vitro sur un dispositif microfluidique composé de deux chambres neuronales qui sont connectées par l'intermédiaire de microcanaux. Les expériences sont réalisées sur une période de temps allant de 2 à 12 jours in vitro (Days in vitro), soit DIV2 à DIV12, et une fixation/acquisition à DIV 12.

Conformément à la Figure 3B, les inventeurs ont traité des neurones avec 1µM de ML-348 quotidiennement à l'intérieur du dispositif microfluidique et l'analyse de transport axonal du BDNF-mCherry a été effectuée dans la partie distale des longs microcanaux.

Sur la Figure 3C, les kymographes montrent le trafic axonal de BDNF-mCherry obtenu à partir de cellules de type sauvage et de cellules Hdh^{CAG/+} traitées ou non par ML-348. On peut observer le défaut de transport axonal de BDNF chez les cellules Hdh^{CAG/+} non traitées, lequel transport étant restauré au moins en partie par le traitement par ML-348 pour atteindre sensiblement celui observé pour les cellules témoins de type sauvage non traitées.

De manière similaire, sur les Figures 3D à 3G, on peut observer que les défauts de transport axonal dans les neurones Hdh^{CAG/+} (maladie de Huntington) se répercutant sur les vitesses de transport antérograde et rétrograde du BDNF-mCherry, le nombre de vésicules antérogrades et rétrogrades se déplaçant sur une distance de 100µm et ceci pendant 30 secondes ainsi que le flux net linéaire sont restaurés, et l'on retrouve des valeurs sensiblement égales à celles de type sauvage lors du traitement du réseau Hdh ^{CAG/+} par ML-348.

Sur la Figure 3H, on peut voir la zone d'enregistrement qui a été choisie pour l'évaluation de la densité de synapses par la quantification du nombre de points de synaptophysine (SYP), un marqueur présynaptique, et du nombre de points de PSD95, un marqueur postsynaptique. La Figure 3I témoigne que dans le réseau microfluidique Hdh^{CAG/+}, le nombre de synapses est diminué dans les dispositifs microfluidiques. Le traitement par ML-348 du réseau de type sauvage a légèrement réduit la connectivité de la synapse, mais ce résultat n'est statistiquement pas significatif. En revanche, le traitement par ML-348 du réseau Hdh^{CAG/+} a permis la restauration du nombre de synapses se rapprochant de celui du réseau de type sauvage. Le double marquage immunofluorescent de SYP et PSD95 permet de visualiser que la colocalisation des marqueurs à l'intérieur de la microchambre est restaurée dans le réseau Hdh^{CAG/+} traité par ML-348. Ceci est indicatif d'une restauration du nombre de synapses par ML-348 dans le réseau Hdh^{CAG/+} (données non illustrées).

Sur la Figure 3J, on peut visualiser la zone d'enregistrement qui a été choisie pour l'analyse du compartiment postsynaptique en situation de stimulation des neurones corticaux par la glycine/strychnine pendant 15 minutes.

La Figure 3K est une représentation graphique du pourcentage de cellules phosphoERK immunopositives dans l'espace postsynaptique. Le traitement par ML-348 de neurones Hdh^{CAG/+} a permis de restaurer la signalisation postsynaptique vers des valeurs rencontrées dans les neurones de types sauvages. Le double marquage pERK/ coloration DAPI permet de visualiser la forte inhibition du marquage pERK dans le réseau Hdh^{CAG/+} non traité, inhibition qui est levée par le traitement par ML-348 qui restaure le marquage vers des valeurs équivalentes à celles du réseau de type sauvage (données non illustrées).

Les résultats précédents ont donc permis de mettre en évidence que l'inhibiteur de APT1, ML-348, restaure les événements présynaptiques, synaptiques et postsynaptiques qui sont altérés dans le réseau cortico-striatal dans la maladie de Huntington, confirmant l'intérêt thérapeutique majeur de cette molécule.

La Figure 3L permet de visualiser au moyen d'un test MTT au sel de tétrazolium MTT (bromure de 3-(4,5-dimethylthiazol-2-yl)-2,5-diphenyl tetrazolium) l'effet nontoxique d'un traitement par ML-348 sur les cellules neuronales du cortex de souris. Ainsi, pour des concentrations de ML-348 de 100 nM, 1µM ou 10µM, aucun effet n'a été observé sur la viabilité des cellules. Seule la concentration de 100µM s'est révélée toxique sur une période de 48 heures, et a été écartée. Le traitement des cellules neuronales par 1µM de ML-348 n'a révélé aucune toxicité, et ce pendant 12 jours.

Sur la Figure 3M, les résultats du test MTT sur des neurones du cortex de souris en culture après un traitement chronique jusqu'à DIV12 indiquent que la concentration utilisée de ML-348 (1µM) n'est pas toxique pour les neurones. En effet, le dosage de la densité optique à 595-690 nm par spectroscopie indique que la quantité relative de neurones vivants et métaboliquement actifs est sensiblement la même entre les témoins (CTRL1 et CTRL2) et les cellules traitées par ML-348.

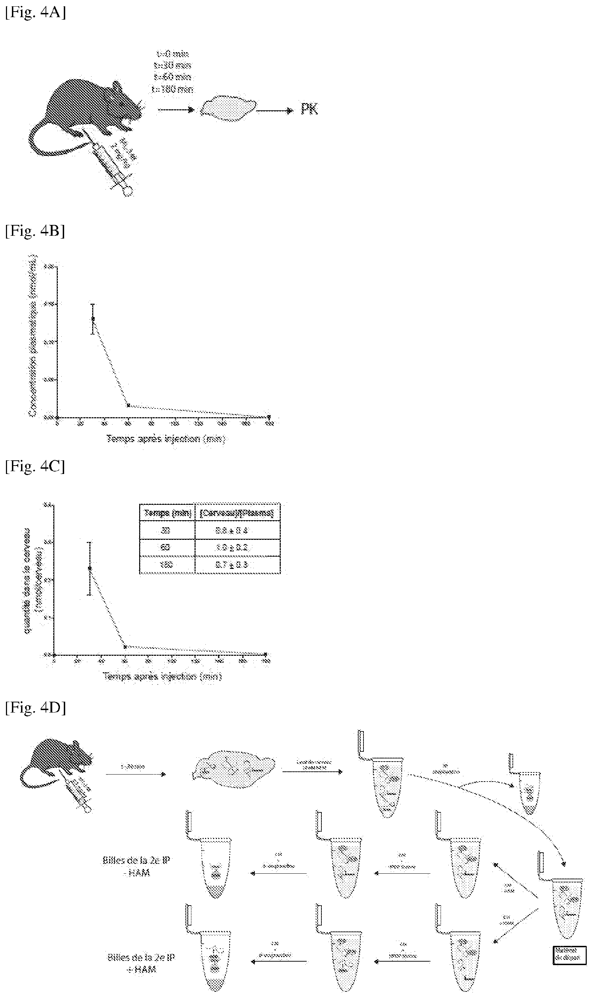

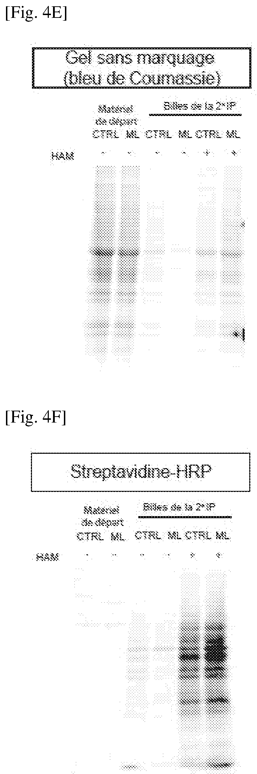

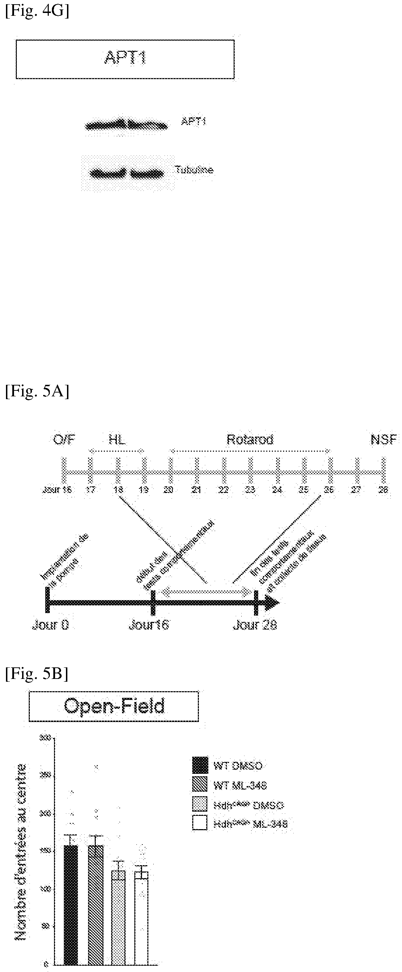

La Figure 4 met en évidence que ML-348 est fonctionnel après l'injection.

Si l'on se réfère à la Figure 4A, les inventeurs ont procédé à l'injection des souris par voie intrapéritonéale avec ML-348 (2 mg/kg) et ont analysé la pharmacocinétique de la molécule. Ils ont mesuré par LC-MS/MS après 30, 60 et 180 minutes la concentration plasmatique (Figure 4B) et la quantité dans le cerveau (Figure 4C) de ML-348. Cette expérience a permis de mettre en évidence que ML-348 traverse efficacement la barrière hémato-encéphalique comme le montrent les valeurs [Cerveau]/[Plasma] dans le tableau de la Figure 4C. Par ailleurs, la molécule est rapidement dégradée 60 minutes après l'injection intrapéritonéale. La capacité de la molécule ML-348 à traverser librement la barrière hémato-encéphalique constitue un argument supplémentaire à l'efficacité de la molécule in vivo chez la souris.

Sur la Figure 4D, on peut visualiser la représentation graphique de l'expérimentation mise en place pour tester l'efficacité de la molécule pour augmenter le taux de palmitoylation dans le cerveau 30 minutes après l'injection. Un test ABE (Acyl-Biotin-Exchange) a été réalisé sur un lysat de cerveau provenant de souris injectées avec une solution de ML-348 à 0,3 mg/kg (ou une solution témoin): les protéines palmitoylées sont d'abord biotinylées avant d'être immunoprécipitées par des billes revêtues de streptavidine.

En conséquence, on peut visualiser par comparaison entre un gel sans marquage (Figure 4E) et un gel en présence de streptavidine-HRP (Figure 4F) que le taux de palmitoylation dans les lysats de cerveau était accru. En revanche, le gel de la Figure 4G indique que le taux d'expression de l'enzyme APT1 n'était pas différent entre les lysats de cerveaux témoins non traités et ceux traités par ML-348 (comparaison avec le taux d'expression de la tubuline).

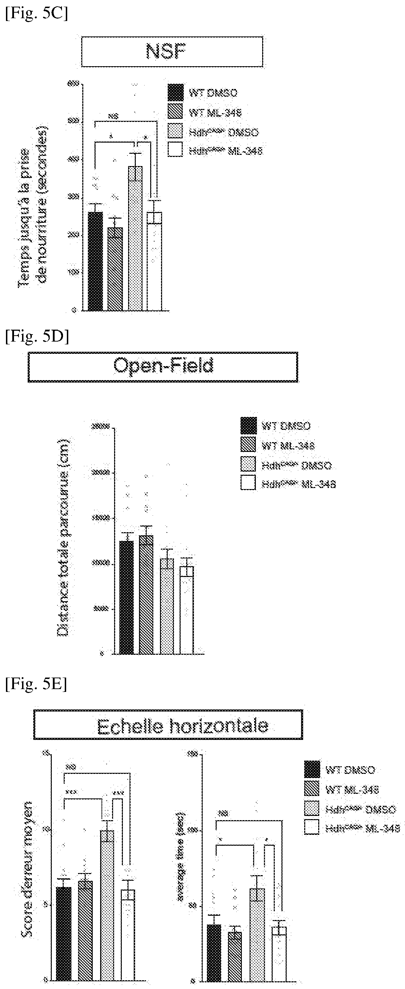

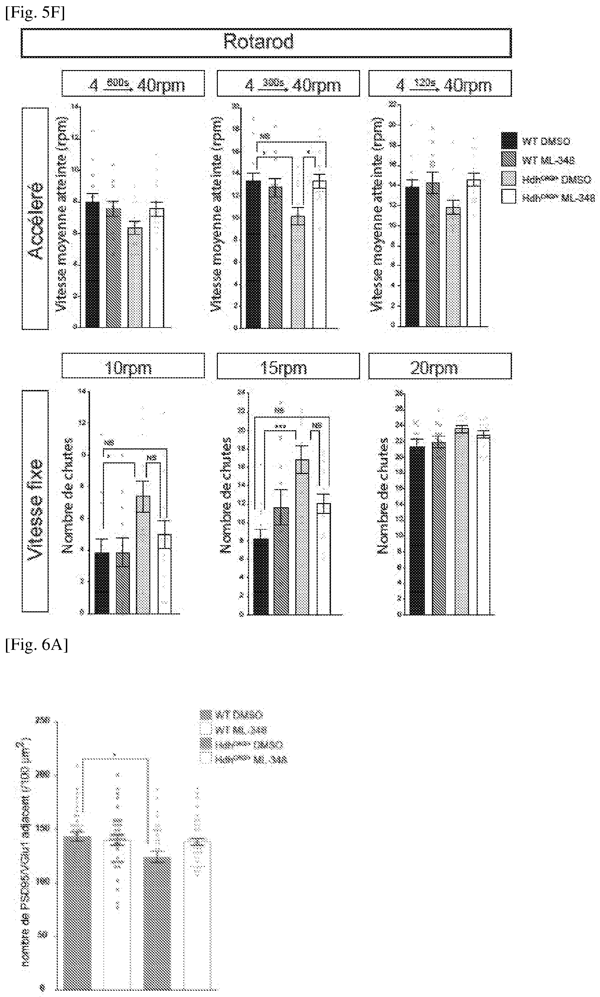

La Figure 5 montre les effets du traitement par ML-348 sur les comportements anxiodépressif (5B-5C) et moteur (5D-5F) chez des souris modèles de la maladie de Huntington (HdhCAG140/+) vs des souris de type sauvage (WT).

Si l'on se réfère à la Figure 5A, on peut visualiser les différents tests comportementaux réalisés sur des souris âgées de 7 mois entre 16 et 28 jours post-implantation d'une micropompe sous-cutanée (traitement chronique par injection sous-cutanée constante de ML-348). Le programme expérimental comprend les tests suivants : O/F: Open Field, HL: Horizontal Ladder, Rotarod et NSF: Novelty-Suppressed Feeding.

Dans un premier temps, sur la Figure 5B, les résultats du test de l'Open Field (champ ouvert) sont exprimés en nombres d'entrées dans la zone centrale sur une période de 60 minutes. Cet essai est établi pour tester les comportements liés à l'anxiété. Les inventeurs ont constaté que le nombre d'entrées au centre de la zone était légèrement inférieur chez les souris Hdh^{CAG/+} par comparaison aux souris de type sauvage. Cependant, ces différences ont été jugées non significatives statistiquement, ce qui présume que le phénotype anxieux lié à la maladie de Huntington n'est pas détectable à 7 mois chez les souris. Le traitement par ML-348 n'a également pas eu d'effet significatif quel que soit le groupe de souris.

Sur la Figure 5C, les inventeurs ont étudié les comportements liés à l'anxiété et la dépression à l'aide du test NSF (test de l'alimentation en environnement nouveau après privation de nourriture) qui est révélateur des stades anxieux et dépressifs. La latence jusqu'à la prise de nourriture dans un nouvel environnement était significativement plus longue pour les souris Hdh^{CAG/+} par comparaison aux souris de type sauvage et le traitement par ML-348 permet de rétablir le comportement des souris Hdh^{CAG/+} vers celui des souris de type sauvage. Ces observations suggèrent que ML-348 a un effet significatif sur les comportements anxio-dépressifs induits par la maladie de Huntington.

En addition aux tests se focalisant sur les comportements anxio-dépressifs, des tests supplémentaires ont été effectués sur les souris pour évaluer leurs comportements moteurs (Figures 5D-5F). Sur la Figure 5D, le graphique représente la locomotion spontanée des souris en tant que distance totale parcourue pendant 60 minutes dans le cadre du test d'Open Field. On constate que la distance parcourue était légèrement inférieure pour les souris Hdh^{CAG/+} par comparaison aux souris de type sauvage, mais cette différence n'a pas été estimée statistiquement significative.

La Figure 5E représente le score d'erreurs moyen et la durée moyenne de traversée de l'échelle du test d'Horizontal Ladder (échelle horizontale). Ce test permet d'étudier la coordination motrice des pattes avant et arrière des souris. On peut noter que les souris Hdh^{CAG/+} ont montré des défauts significatifs dans cette tâche comme en témoignent les scores d'erreurs élevés et l'augmentation de la durée de traversée de l'échelle. Une amélioration des performances des souris est rendue possible par le traitement par ML-348.

La Figure 5F représente, sur le panel du haut, la vitesse moyenne atteinte (en rotations par minute, rpm) par les souris sur un appareil rotarod à vitesse accélérée, de 4 à 40 rpm en 600 secondes, de 4 à 40 rpm en 300 secondes et de 4 à 40 rpm en 120 secondes, respectivement. On observe que les souris Hdh^{CAG/+} traitées par ML-348 retrouvent une coordination motrice proche de celle des souris de type sauvage, en particulier dans la condition 300s où les résultats ont été jugés statistiquement significatifs. Dans le panel du bas, la vitesse moyenne atteinte par les souris est étudiée sur un appareil rotarod fixe à vitesse constante de, respectivement, 10 rpm, 15 rpm et 20 rpm. Une vitesse élevée en rotarod à vitesse fixe (20 rpm) ne permet pas de montrer de différence significative entre les souris de type sauvage et les souris Hdh^{CAG/+}, en revanche, à une vitesse moindre (10 et 15 rpm), ML-348 a amélioré significativement les performances des souris Hdh^{CAG/+} puisque les valeurs observées étaient similaires à celles des souris de type sauvage non traitées.

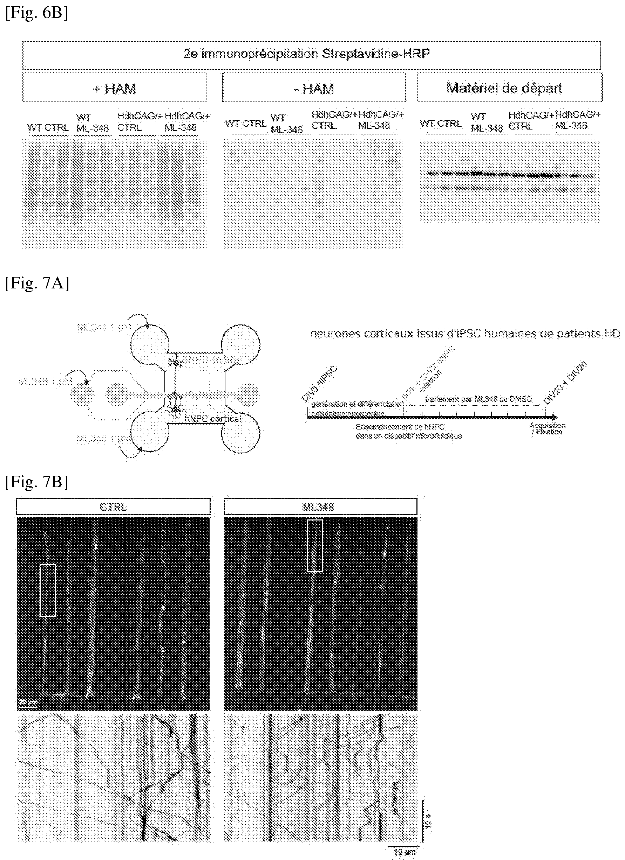

La Figure 6 montre l'augmentation de la densité de synapses et de la palmitoylation après un traitement par ML-348 de 28 jours.

La Figure 6A permet de chiffrer la densité de synapses post-mortem sur des neurones de souris témoins ou après 28 jours de traitement par ML-348 au moyen du nombre de points adjacents de PSD95 et Vglut-1 pour 100 µm². On note qu'il y a une réduction significative de ce nombre de points adjacents chez les souris Hdh^{CAG/+} non traitées. Le traitement par ML-348 restaure la densité synaptique à un niveau sensiblement égal à celui des souris sauvages non traitées. (WT CTRL=36; WT ML-348=36; HdH^{CAG/+} CTRL=36; HdH^{CAG/+} ML-348=36; F3,140 =3.65 , * p<0.05)

La Figures 6B représente les résultats d'un essai ABE sur des immunoprécipitats de cerveaux de souris WT et HdH^{CAG/+}, avec ou sans traitement par ML-348. Après la seconde immunoprécipitation avec des billes revêtues de streptavidine-HRP, on peut constater, par comparaison des bandes obtenues sur gel en présence d'hydroxylamine (+HAM) et en son absence (-HAM), que le traitement par ML-348 sur les souris HdH ^{CAG/+} augmente bien la palmitoylation. En effet, une protéine est présumée palmitoylée si elle est détectée par blotting avec streptavidine-HRP dans la condition +HAM mais si aucun signal spécifique n'est détectable dans la condition -HAM.

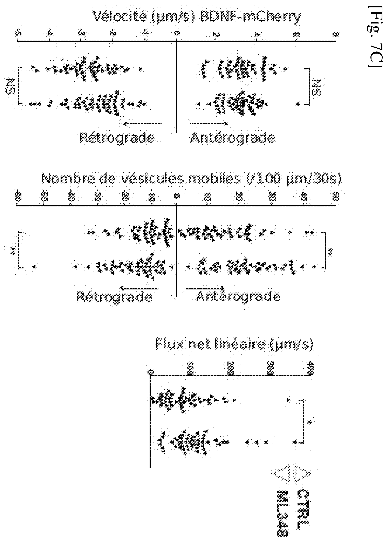

La Figure 7 montre l'effet du traitement par ML-348 sur le trafic de BDNF dans des neurones corticaux issus de cellules souches pluripotentes induites (iPSC) humaines provenant de patients atteints de la maladie de Huntington.

Si l'on se réfère à la Figure 7A, les inventeurs ont de nouveau reproduit le réseau cortico-striatal in vitro sur un dispositif microfluidique composé de deux chambres neuronales qui sont connectées par l'intermédiaire de microcanaux. Les expériences sont réalisées sur une période allant de 0 à 40 jours in vitro (DIV), les 20 premiers jours pour la génération et la différenciation cellulaires neuronales des hiPSC, puis l'introduction des cellules dans le dispositif microfluidique. 3 heures après l'introduction, les cellules ont été infectées à partir du compartiment pré-synaptique par un lentivirus codant pour BDNF-mCHerry. Après 7 à 10 jours de culture, la formation de contacts cortico-corticaux ont été observés dans le compartiment synaptique, générant un circuit cortico-cortical humain typique de la maladie de Huntington. 13 jours après l'introduction, les compartiments présynaptique, synaptique et postsynaptique des microchambres ont été traités par 1 µM de ML-348 quotidiennement pendant 7 jours.

Sur la Figure 7B, les enregistrements et leurs kymographes montrent le trafic axonal de vésicules de BDNF-mCherry dans la partie distale des microcanaux présynaptiques qui contiennent uniquement des axones corticaux. On peut observer le défaut de transport axonal de BDNF chez les cellules traitées par le DMSO, lequel transport étant restauré au moins en partie par le traitement par ML-348.

De manière similaire, sur la Figure 7C, on peut observer que les défauts de transport axonal dans les neurones corticaux issus d'iPSC humaines (maladie de Huntington) se répercutent sur les vitesses de transport antérograde et rétrograde du BDNF-mCherry, le nombre de vésicules antérogrades et rétrogrades se déplaçant sur une distance de 100µm et ceci pendant 30 secondes ainsi que le flux net linéaire. Les résultats obtenus pour les vitesses de transport antérograde et rétrograde des vésicules de BDNF-mCherry ne se sont pas révélés statistiquement significatifs (N= nombre d'axones par condition dans au moins deux expériences indépendantes : N=47 témoins, N=52 ML-348 ; antérograde : p=0,4892, rétrograde : p=0,1776 selon le test t). En revanche, le nombre de vésicules se déplaçant dans l'une et l'autre des directions (antérograde : p=0,0068, rétrograde : p=0,0026, Mann-Whitney **p<0,01) et le flux net linéaire (p=0,0313, Mann-Whitney *p<0,05) ont été significativement augmentés par le traitement par ML-348.

### EXEMPLES

Les exemples suivants illustrent l'invention.

### Matériel et Méthodes

### Animaux

Les expériences suivantes sont réalisées sur des souris Hdh^{CAG140/+} knock-in qui sont générées sur un fond génétique C57-BL6J et expriment la séquence de l'exon 1 de la Huntingtine HTT humaine (SEQ ID NO :1)avec 140 répétitions de CAG. Les souris de type sauvage WT et les souris mutantes Hdh^{CAG140/+} knock-in ont été maintenues avec un accès à la nourriture et à l'eau *ad libitum* et conservées à température constante (19-22°C) et humidité constante (40-50%) sur un cycle 12h:12h lumière/obscurité. Toutes les procédures expérimentales ont été réalisées dans une installation autorisée respectant les recommandations de la Communauté Economique Européenne (Directive 86/609/EEC) et le Comité National Français (transposition de la directive européenne 2010-63UE) pour les soins et l'utilisation d'animaux de laboratoire sous la supervision de chercheurs autorisés.

### Cellules modèles de la maladie de Huntington

Les cellules *StHdh* ^{*Q111*/*Q111*} sont des cellules striatales immortalisées à partir de souris *Hdh* ^{*Q111*/*Q111*} . Ce modèle de souris est un modèle knock-in qui exprime la séquence de l'exon 1 de la Huntingtine HTT humaine (SEQ ID NO :1) avec 111 répétitions de CAG.

### Système RUSH

Le plasmide RUSH (Boncompain et al., 2012) est un plasmide bicistronique (SEQ ID NO : 2) permettant l'expression de deux parties du système : l'ancre spécifique du réticulum (KDEL-streptavidine), et la partie rapporteur (GPI-SBP-mCh). L'ajout de la biotine dans le milieu extracellulaire (40µM) induit le décrochage de la partie rapporteur de l'ancre, permettant ainsi de suivre le trafic intracellulaire de manière synchronisée au sein d'une population cellulaire depuis le réticulum endoplasmique.

### Traitements

ML-348 (Tocris-5345), Palmostatine B (Merck Millipore-178501) et ML-349 (obtenu auprès de Martin Brent) ont été dilués dans 100% DMSO pour la concentration primaire.

Pour les cellules striatales *StHdh,* un traitement aigu a été réalisé à 10µM pendant 1 heure avant et pendant le temps d'acquisition. Pour les neurones au sein des microchambres, les traitements ont été démarré à DIV2 jusqu'à DIV12 chaque jour à 1µM dans chaque compartiment du dispositif microfluidique.

Chez les souris, une perfusion de ML-348 ou de la solution témoin (solution saline + DMSO) pendant 28 jours à 0,3 mg/kg/jour a été réalisée à l'aide d'une pompe osmotique (mini pompe osmotique de Alzet, 2004) implantée de manière sous-cutanée sur le dos de la souris.

### Tests comportementaux

Les portées de souris ont été regroupées en des groupes de traitements mixtes. Les mâles et les femelles étaient âgés de 6 mois au début du traitement. Pour chaque test spécifique, tous les groupes expérimentaux ont été testés le même jour, pendant la phase de lumière et entre les Jours 16 et 28 de traitement. Avant le test, les souris étaient acclimatées à la pièce de test pendant une période de 30 minutes. Les traitements ont été attribués de manière aléatoire et les tests comportementaux ont été réalisés 12 jours avant la fin de l'administration du traitement. Quatre groupes expérimentaux ont été définis : WT CTRL (témoins de type sauvage : 13 souris : 7 mâles et 6 femelles), WT ML-348 (souris de type sauvages traitées par ML-348 : 12 souris : 7 mâles et 5 femelles), Hdh^{CAG/+}CTRL (souris témoins Hdh^{CAG/+}: 14 souris : 6 mâles et 8 femelles) et Hdh^{CAG/+} ML-348 (souris Hdh^{CAG/+} traitées par ML-348 : 15 souris : 6 mâles et 9 femelles). Les souris ont été exclues de l'analyse si leur performance a été calculée comme étant aberrante par le test de Grubb. Une souris a été exclue selon ces critères. Le poids des souris a été mesuré avant le début du traitement et jusqu'à la fin des tests.

### Open Field test (test de champ ouvert)

La locomotion spontanée a été mesurée comme la distance totale parcourue et l'anxiété générale comme le nombre d'entrées dans la zone interne. Le test Open Field a été réalisé dans une chambre carrée en Plexiglas opaque (50x50 cm) et la chambre interne était définie par une forme quadrangulaire de 12,50 x 12,50 cm. Les souris ont été filmées pendant 6 minutes. La distance parcourue et le nombre d'entrées dans la chambre interne par intervalle de 5 minutes ont été mesurés à l'aide du logiciel de suivi ViewPoint.

### Test Rotarod

L'appareil Rotarod a été utilisé pour mesurer la coordination motrice et l'équilibre. Le test Rotarod a été réalisé sur 6 jours consécutifs avec 3 jours de rotarod accéléré puis 3 jours de rotarod fixe. Pour le rotarod accéléré, le premier jour, les souris ont été soumises à 3 essais à des vitesses croissantes de 4 rpm (rotation par minute) à 40 rpm sur 600 sec, le deuxième jour, les souris ont été soumises à 3 essais à des vitesses croissantes de 4 rpm à 40 rpm sur 300 sec et le troisième jour, les souris ont été soumises à 3 essais à des vitesses croissantes de 4 rpm à 40 rpm sur 120 sec. Chaque essai a été suivi par 5 minutes de repos. La latence et la vitesse à laquelle les souris chutent du rotarod ont été mesurées jusqu'à 300 secondes. Pour un rotarod fixe, les souris ont été évaluées pendant 3 essais à 10, 15 et 20 rpm aux jours 4, 5 et 6 respectivement. Durant 300 sec, les animaux ont été reposés sur le rotarod à chaque fois qu'ils tombaient. Chaque essai a été séparé par une période de 15 minutes de repos. La latence jusqu'à la première chute et le nombre de chutes ont été rapportés. Les données sont exprimées sous la forme de la moyenne des 3 essais.

### Horizontal ladder test (test de l'échelle horizontale)

Une échelle horizontale est utilisée pour évaluer le placement et la coordination des pattes avant et des pattes arrière. L'échelle horizontale est composée de deux parois de Plexiglas transparentes (69,50 x 15 cm) contenant des échelons métalliques (0,2 cm de diamètre) espacés de manière irrégulière (entre 0,5 et 2 cm de distance). Les souris ont été habituées à marcher sur une échelle horizontale pendant deux jours consécutifs (3 essais par jour). Puis les souris sont testées (3 essais) avec un motif différent des deux jours d'entraînement. Les essais de test sont filmés et la performance motrice ainsi que la latence pour compléter la tâche sont quantifiées. Les données sont exprimées sous la forme de la moyenne des 3 essais.

### Novelty-suppressed feeding test (test d'alimentation en environnement nouveau après privation de nourriture)

Le test a été réalisé conformément à la description de Pla et al., 2013. Les animaux ont été privés de nourriture pendant 24 heures pour augmenter leur motivation à s'alimenter. Le jour du test, les souris ont été placées à l'intérieur de l'appareil de test qui consiste en une boîte en plastique (50 cm x 30 cm x 15 cm), dont le fond est recouvert de copeaux de bois. Les souris ont été filmées et laissées explorer librement l'espace jusqu'à ce qu'elles mangent la pastille de nourriture ou pendant une durée maximale de 10 minutes. On a considéré que les souris ont mangé la pastille lorsqu'elles ont mordu la pastille en s'asseyant confortablement.

### Test ABE (Acyl-Biotin-Exchange)

Les cerveaux ont été lysés de manière mécanique dans un tampon glacé (1% IGEPAL CA-630 ; 50mM Tris-HCl pH 7,5, 150mM NaCl, 10% glycérol) contenant 1:100 d'un cocktail d'inhibiteurs de protéases (Sigma Aldrich P8340), 1mM PMSF, 1:200 d'inhibiteur de phosphatase (Sigma Aldrich P5726) et 50mM de NEM (Sigma Aldrich E3876) pour bloquer les cystéines libres. Les lysats de cerveau ont été mis en agitation à 4°C pendant 30 minutes avant que le matériel insoluble soit retiré par centrifugation à 15000 rpm pendant 10 minutes. Les lysats ont été préclarifiés par incubation avec des billes Dynabeads M-280 Streptavidine (Invitrogen 112.05D) pendant 30 minutes à 4°C sur la roue. Les protéines provenant des échantillons préclarifiés ont ensuite été précipitées à l'aide d'un dosage au chloroforme-méthanol. Chaque culot a été divisé et remis en suspension dans +HAM (2:3 du culot) (tampon de lyse contenant 1mM de HAM (Sigma 46780-4)) ou -HAM (1:3 du culot) pendant 1 heure à température ambiante sur la roue. Les protéines ont été précipitées par la méthode au chloroforme-méthanol et les culots ont été remis en suspension dans un tampon Biotine-BMCC (tampon de lyse avec 5µM de EZ-Link^{™} Biotine-BMCC (Thermo Fisher 21900)) pendant une heure à température ambiante sur la roue. Ensuite, les protéines ont encore été précipitées par la méthode au chloroforme-méthanol et les culots ont été remis en suspension dans le tampon de lyse. Les protéines palmitoylées ont été immunoprécipitées avec les billes Dynabeads M-280 Streptavidine (Invitrogen 112.05D) pendant 30 minutes à 4°C avec agitation. Les protéines ont été éluées par ébullition des échantillons avec un tampon d'élution (2,5% de SDS, 2,5% de glycérol, 62,5mM de Tris HCl pH 6,8, 10,005% de bleu de bromophénol et 5mM de DTT). Les échantillons ont été chargés sur un gel à 12% d'acrylamide puis électrophorèse SDS-PAGE et transfert sur nitrocellulose. Le gel Stain Free sans marquage a révélé le témoin de charge et la palmitoylation a été détectée à l'aide de streptavidine conjuguée à HRP 1:10000 (Thermo Fisher Scientific 21126). Pour détecter le taux d'APT1 les anticorps primaires utilisés étaient : anti-APT1 1:1000 (Abcam ab91606) et anti-α-tubuline 1:1000 (Sigma Aldrich T9026).

### Analyse par spectrométrie de masse

Les protéines du plasma ont été précipitées par ajout de 800µL d'acétonitrile dans 400µL d'échantillon. Les cerveaux ont été broyés dans 400µL d'H₂0 avant l'ajout de 800µL d'acétonitrile. Les échantillons ont été centrifugés pour éliminer les résidus insolubles à 15000 g pendant 5 minutes à 16°C. Les surnageants ont été analysés par LC-MS/MS. LC-MS/MS a été analysé par chromatographie liquide à haute performance (UHPC) couplée à un quadrupole triple (Shimadzu LC-MS 8030). Les analyses ont été réalisées à partir de 3 souris pour chaque temps.

### Essai de toxicité

La toxicité a été évaluée à l'aide du test MTT au sel de tétrazolium. La culture corticale a été traitée chaque jour comme décrit plus haut. Après le traitement, une solution de MTT (Life M6494) a été ajoutée au milieu de culture à 1,2mM et incubée pendant 3 heures. La réaction a été stoppée par l'ajout de la solution de solvant (4mM HCl, 0,1% de NP40 dans de l'isopropanol). Les valeurs d'absorbance ont été mesurées à 595 et 690 nm.

### Génération de précurseurs de neurones corticaux à partir de cellules souches pluripotentes induites humaines (hiPSC)

La lignée ND42222 (RRID:CVCL_Y844 passage 42) « 109Q » d'iPSC humaines a été obtenue auprès de Coriell Cell Repositories. Cette lignée est hétérozygote pour HTT p.Gln18[109] et par conséquent présente 109 répétitions CAG dans l'un des deux allèles de *HTT.* L'amplification des iPSC humaines, la génération des cellules neuronales et la différenciation terminale ont été réalisées selon les travaux publiés dans Gribaudo S. et al. (Stem Cell Reports 12, 230-244, 2019). Les cellules 109Q ont été maintenues sur des plaques revêtues de Vitronectine (Life Technology) dans un milieu mTeSRplus (STEMCELL Technologies). Les cultures ont été alimentées un jour sur deux et passées par dissociation manuelle à l'aide de 0,02% d'EDTA pH 7,2 (Merck Sigma-Aldrich) tous les 4-5 jours. Pour la différenciation neuronale, les colonies de hiPSC ont été traitées (DIV0) tel que décrit précédemment (Nicoleau et al., 2013) dans un milieu N2B27 consistant en 50% DMEMF-12 Glutamax, 50% milieu neurobasal, 2% supplément B27 50x sans vitamine A, 1% supplément N2, 0,1% pénicilline streptomycine et 50µM β-mercaptoéthanol (Thermofisher). La différenciation neuronale a été initiée par le passage des hiPSC dans un milieu N2B27 supplémenté en SB431542 (20µM, Tocris), LDN-193189 (100 nM, Sigma Aldrich) et XAV_939 (1µM, Tocris) et 10 µM d'inhibiteur ROCK (Y27632, Calbiochem) sur des plaques de culture à faible adhérence (Greiner) pendant 6 heures. Le milieu a été changé tous les jours de DIV0 à DIV20. A DIV1, les agrégats de hiPSC ont été transférés sur des boîtes revêtues de poly-ornitine laminine sans Y27632. De DIV5 à DIV9, SB431542 a été retiré et FGF2 (10 ng/ml) et cyclopamine 1µM (Merck) ont été ajoutés. De DIV10 à DIV20, LDN-193189 et XAV-939 ont été retirés et CHIR99021 0,4µM (Stemgent) a été ajouté. A DIV20, les cellules précurseurs de neurones corticaux ont été dissociées enzymatiquement à l'aide d'Accutase (Invitrogen), remises en suspension à 5x10⁶ cellules/ml dans le milieu de cryopréservation cellulaire Cryostor (Merck), congelées et stockées dans de l'azote liquide à -150°C.

### Différenciation neuronale de cellules précurseurs de neurones corticaux issues de hiPSC dans des dispositifs microfluidiques

Les dispositifs microfluidiques ont été revêtus de poly-D-lysine (Merck) dans les compartiments proximal et synaptique et de poly-D-lysine/laminine (Thermofisher) dans le compartiment distal. Les précurseurs de neurones corticaux ont été mis en suspension dans un milieu N2B27 supplémenté en BDNF (20 ng/ml, Preprotech), AMPc (100µM, Merck), DAPT (10µM, Tocris), Cdk4i (1µM, Merck) et l'inhibiteur ROCK (Y-27632, Stemcell technologies) et déposés sur plaques à une densité finale d'environ 7000 cellules/mm² dans le compartiment distal de la chambre microfluidique. Trois heures après le dépôt, les cellules ont été infectées par un lentivirus codant pour BDNF-mCherry. Le jour après l'ensemencement, le milieu a été remplacé par du milieu frais N2B27 supplémenté sans l'inhibiteur ROCK. Le milieu a ensuite été changé tous les 7 jours. Les cellules ont été exposées de DIV+13 à DIV+20 (après l'ensemencement dans le dispositif microfluidique) chaque jour avec 1µM de ML-348 ou DMSO ajouté à la fois dans les compartiments proximal, synaptique et distal du dispositif microfluidique.

### Analyse statistique

Le logiciel GraphPad Prism (GraphPad Software, Inc.) a été utilisé pour l'analyse statistique. Toutes les expériences ont consisté en au moins 3 expériences répliquées indépendantes. Les données sont exprimées en tant que moyenne +/- erreur-type. Les groupes ont été comparés à l'aide d'une analyse à un facteur de la variance, que l'on a fait suivre par une analyse post-hoc de Tukey pour de multiples comparaisons ou deux groupes ont été comparés à l'aide d'un test de Student apparié à deux séries. La distribution des données a été estimée normale. Le critère pour la significativité a été fixé à p<0,05.

### Résultats expérimentaux

** Le trafic du Réticulum endoplasmique (RE) vers la membrane plasmique est altéré dans la maladie de Huntington*

Les inventeurs ont utilisé le système RUSH (Retention Using Selective Hooks) pour étudier les dynamiques intracellulaires du RE vers la membrane plasmique dans la maladie de Huntington (FIG 1A). RUSH permet de suivre la libération synchrone d'une molécule rapportrice à partir de compartiments intracellulaires spécifiques. Dans la présente expérience, les inventeurs ont suivi la libération de l'ancre glycosylphosphatidylinositol (GPI) marqué par la protéine de fluorescence mCherry, et fusionnées à un peptide de liaison à la streptavidine (SBP) qui a été retenu à l'intérieur du compartiment RE par l'interaction avec une protéine « hameçon » (hook) composée du motif KDEL fusionné à la streptavidine. En l'absence de biotine, la GPI-SBP-mCherry se localise au niveau du RE (FIG 1A). L'ajout de biotine aux cellules exprimant GPI-SBP-mCherry et KDEL-streptavidine provoque une libération synchrone de la GPI-SBP-mCherry rapportrice de la protéine « hameçon », qui ensuite migre vers l'appareil de Golgi puis vers la membrane plasmique.

Pour étudier cette dynamique dans le contexte de la maladie de Huntington, les inventeurs ont transfecté la construction GPI-SBP-mCherry-KDEL-streptavidine telle que représentée par SEQ ID NO :2 dans des cellules striatales immortalisées de souris *Hdh* ^{*Q111*/*Q111*} et *Hdh* ^{*Q7*/*Q7*} , appelées respectivement cellules *StHdh* ^{*Q111*/*Q111*} et *StHdh* ^{*Q7*/*Q7*} . Ils ont ensuite suivi le trafic de GPI-SBP-mCherry par imagerie en temps réel pendant 90 minutes. Pour évaluer les dynamiques de trafic intracellulaire, ils ont quantifié les cinétiques d'apparition et de disparition de la fluorescence mCherry dans l'appareil de Golgi au cours du temps (Fig 1 B et C). Peu après l'ajout de biotine, GPI quitte le RE et se déplace vers l'appareil de Golgi. Les inventeurs ont quantifié la fluorescence au niveau du Golgi et ont observé que dans les cellules *StHdh* ^{*Q7*/*Q7*} , le pic de fluorescence était à 16,6 minutes, alors qu'il était retardé jusqu'à 23,6 minutes dans les cellules *StHdh* ^{*Q111*/*Q111*} (FIG 1B). Leurs observations indiquent un trafic réduit de GPI du RE vers l'appareil de Golgi ainsi qu'une rétention prolongée de GPI dans le Golgi comme en témoigne une période de fluorescence plus longue de GPI dans l'appareil de Golgi. Ces résultats sont en accord avec des observations préalables de trafic altéré entre le réticulum endoplasmique et l'appareil de Golgi et entre ce dernier et la membrane plasmique dans la maladie de Huntington.
** L'inhibition d'APT1 restaure le trafic du réticulum endoplasmique vers la membrane plasmique*

Comme indiqué plus haut, dans la maladie de Huntington, il existe une réduction de la palmitoylation de la Huntingtine HTT, ainsi qu'une réduction de l'activité d'enzymes de palmitoylation, HIP14 et HIP14L, suggérant qu'en addition à HTT elle-même, il existe une dérégulation globale de la palmitoylation cellulaire à l'intérieur des neurones lorsque HTT contient l'extension polyQ.

Bien que ces résultats suggèrent que HIP14 et HIP14L puissent être des cibles potentielles d'intérêt thérapeutique, il n'existe pas de molécules pour améliorer l'activité de HIP14. En revanche, des molécules qui inhibent les dépalmitoylases (acyl-thioestérases) ont été développées (FIG 2A).

Les inventeurs ont d'abord testé l'effet de la Palmostatine B sur les cinétiques intracellulaires à l'aide du système RUSH. Ils ont découvert que la Palmostatine B restaure les cinétiques du RE vers l'appareil de Golgi et diminue également la durée que prend la fluorescence pour quitter l'appareil de Golgi (FIG 2B). Cependant, la Palmostatine B n'est pas spécifique d'une APT donnée et elle cible à la fois APT1 et APT2 avec des affinités similaires (CI50_{APT1}=5,4 nM and CI50_{APT2}=37,7 nM). Les inventeurs ont ensuite observé qu'un traitement par ML-348 (inhibiteur de APT1) était capable de restaurer les cinétiques, alors que ML-349 (inhibiteur de APT2) n'avait pas d'effet, ce qui suggère que l'inhibition de APT1 plutôt que APT2 peut restaurer le trafic du réticulum endoplasmique vers la membrane plasmique qui est altéré dans les cellules affectées par la maladie de Huntington (FIG 2 C et D). Ces résultats révèlent que APT1 et non APT2 est spécifiquement mise en jeu dans les mécanismes pathophysiologiques de la maladie de Huntington et indiquent que APT1 est une cible thérapeutique potentielle dans la maladie de Huntington pour la restauration des dynamiques intracellulaires.

Les résultats de l'expérience de silencing génique au moyen de petits ARN interférents dirigés contre APT1 (si-APT1, FIG 2F) et APT2 (si-APT2, FIG 2G), respectivement, contribuent à confirmer que les effets observés grâce aux inhibiteurs ML-348 et ML-349 sur le trafic intracellulaire (FIG 2C et FIG 2D) sont effectivement dus à une inhibition des enzymes responsables de la dépalmitoylation APT1 et APT2.
** L'inhibition de APT1 restaure le réseau cortico-striatal sur une puce*

Les inventeurs ont ensuite étudié si ML-348 est capable de restaurer les dysfonctions du réseau cortico-striatal dans la maladie de Huntington. En effet, le circuit cortico-striatal est particulièrement altéré dans la maladie de Huntington, ce qui se manifeste par une dysfonction et une dégénérescence à la fois des neurones striataux et de leurs afférences cortico-striatales. Ils ont développé une approche microfluidique qui permet la reconstruction d'une manière contrôlée du point de vue spatial et temporel, compatible avec la vidéomicroscopie confocale en fast spinning. Ce système utilise un dispositif microfluidique à base de polymère de silicium composé de deux chambres neuronales isolées du point de vue fluidique, qui sont connectées par l'intermédiaire d'un ensemble de minces microcanaux à travers lesquels les neurites peuvent croître et entrer en contact les uns avec les autres dans un compartiment synaptique intermédiaire. A l'aide de cultures neuronales primaires, ils ont reconstitué un réseau cortico-striatal dans lequel des neurones corticaux se projettent vers des neurones striataux cibles par l'intermédiaire de connexions axodendritiques orientées (FIG 3A). Ces dispositifs sont compatibles avec le traitement médicamenteux et par conséquent peuvent être utilisés pour tester des agents pharmacologiques *in vitro* sur ce circuit fonctionnel.

En premier lieu, les inventeurs ont testé la toxicité du traitement par ML-348 sur des structures corticales de type sauvage à 1µM pendant 10 jours. Cet essai n'a pas révélé de toxicité à cette concentration (CI50_{APT1} = 230 nM) (FIG 3M).

Les inventeurs ont ensuite traité des neurones avec 1µM de ML-348 quotidiennement à l'intérieur du dispositif microfluidique et ont mesuré les dynamiques des vésicules contenant du BDNF dont le trafic est perturbé dans la maladie de Huntington. Les neurones corticaux des souris WT et Hdh^{CAG/+} ont été infectés avec un lentivirus BDNF-mCherry et des images ont été prises au Jour 12 dans la partie distale des longs microcanaux pour analyser le transport axonal du BDNF (FIG 3B). Comme prévu, des défauts de transport axonal dans les neurones MH (maladie de Huntington) ont été identifiés à la fois dans les vitesses de transport antérograde et rétrograde, et aussi dans le nombre de vésicules se déplaçant le long des microtubules (FIG 3C-3E). Ces effets ont conduit à une réduction du flux net linéaire sans impact sur la directionnalité des vésicules (FIG 3F et 3G). Les inventeurs ont observé que le traitement de neurones WT par ML-348 avait un effet inhibiteur sur le trafic du BDNF d'une manière similaire à celui qui est observé dans les neurones Hdh^{CAG/+}. A contrario, ils ont observé que le traitement de neurones Hdh^{CAG/+} par ML-348 restaurait tous les paramètres de transport, notamment les vitesses de transport antérograde et rétrograde, le nombre de vésicules, le flux net linéaire vers des valeurs témoins de type sauvage.

Les inventeurs ont ensuite évalué la densité de synapses par quantification du nombre de points de synaptophysine (un marqueur présynaptique, SYP) adjacent à des points de PSD95 (un marqueur postsynaptique) à l'intérieur de la chambre synaptique du dispositif microfluidique à l'aide de la microscopie confocale Airyscan à haute résolution (FIG 3H). Dans le réseau Hdh^{CAG/+}, le nombre de synapses est diminué dans les dispositifs microfluidiques (FIG 3I). Le traitement par ML-348 du réseau WT a légèrement réduit la connectivité de la synapse. Cependant, ceci n'était pas significatif. D'une manière importante néanmoins, le traitement par ML-348 du réseau Hdh^{CAG/+} a restauré le nombre de synapses vers celui du réseau de type sauvage WT (FIG 3I). Le double marquage immunofluorescent de SYP et PSD95 a également permis de visualiser que la colocalisation des marqueurs à l'intérieur de la microchambre est restaurée dans le réseau HdhCAG/+ traité par ML-348. Ceci est indicatif d'une restauration du nombre de synapses par ML-348 dans le réseau HdhCAG/+ (données non illustrées).

Pour finir, les inventeurs ont déterminé les conséquences du traitement par ML-348 sur la signalisation de survie dans le compartiment postsynaptique. Ils ont utilisé la phosphorylation de ERK comme indice de la signalisation postsynaptique. En effet, plusieurs études ont montré des défauts de signalisation pERK dans le striatum dans les conditions de MH. Les inventeurs ont stimulé des neurones corticaux pendant 15 minutes avec glycine/strychnine et ont quantifié le pourcentage de neurones striataux immunopositifs pour phosphoERK (FIG 3J). Le traitement par ML-348 de neurones Hdh^{CAG/+} a restauré la signalisation postsynaptique vers des valeurs rencontrées dans les neurones de type sauvage (FIG 3K). Par ailleurs, un double marquage pERK/ coloration DAPI a permis de visualiser la forte inhibition du marquage pERK dans le réseau HdhCAG/+ non traité, inhibition qui est levée par le traitement par ML-348 qui restaure le marquage vers des valeurs équivalentes à celles du réseau de type sauvage (données non illustrées).

Toutes ces observations indiquent que l'inhibiteur ML-348 de APT1 restaure les événements présynaptique, synaptique et postsynaptique qui ont été altérés dans le réseau cortico-striatal dans la maladie de Huntington, suggérant que cette molécule a un effet thérapeutique d'intérêt in vivo.
** Le traitement par ML-348 augmente la palmitoylation dans le cerveau*

Avant d'étudier ML-348 *in vivo,* les inventeurs ont d'abord évalué la pharmacocinétique de la molécule. Ils ont injecté ML-348 à des souris par voie intrapéritonéale et ont mesuré par LC-MS/MS après 30, 60 et 180 minutes la concentration plasmatique et la quantité dans le cerveau de ML-348 (FIG 4A, 4B et 4C). Ils ont découvert que ML-348 traverse efficacement la barrière hémato-encéphalique comme le montrent les valeurs du rapport [Cerveau]/[Plasma] (FIG 4C, tableau). Cependant, la molécule était rapidement dégradée 60 minutes après l'injection péritonéale (FIG 4B et 4C). Ils ont ensuite évalué l'efficacité de la molécule pour augmenter le taux de palmitoylation dans le cerveau 30 minutes après l'injection. Pour cela, ils ont réalisé un essai ABE (Acyl-Biotin-Exchange) pour biotinyler des protéines palmitoylées avant leur immunoprécipitation avec des billes revêtues de streptavidine (FIG 4D). Après l'injection du traitement par ML-348, ils ont observé que le taux de palmitoylation dans le cerveau était accru alors qu'il n'y avait pas de modification de l'expression d'APT1 (FIG 4E-4G). Ces résultats indiquent que ML-348 traverse facilement la barrière hématoencéphalique et augmente efficacement la palmitoylation dans le cerveau, suggérant que la molécule soit efficace chez la souris in vivo.
** ML-348 chronique améliore les déficiences cognitives chez les souris Hdh* ^{*CAG*/*+*}

Les inventeurs ont ensuite évalué les effets d'une perfusion constante de ML-348 pendant 28 jours sur le comportement de la maladie de Huntington (FIG 5A). D'abord, ils ont évalué les conséquences d'un traitement chronique sur des tests comportementaux non moteurs (FIG 5B-5C). En effet, les modifications neuropsychiatriques sont prévalentes parmi les patients atteints de la maladie de Huntington et celles-ci ont souvent lieu avant l'apparition des symptômes moteurs.

Les inventeurs ont utilisé le protocole Open Field OF (champ ouvert) pour tester les comportements liés à l'anxiété. Ils ont observé que le nombre d'entrées au centre de la zone était légèrement inférieur chez les souris Hdh^{CAG/+} par comparaison aux souris de type sauvage. Cependant, les différences n'étaient pas significatives, ce qui suggère que le phénotype anxieux n'est pas détectable à 7 mois (FIG 5B). Le traitement par ML-348 n'a également pas eu d'effet significatif sur les souris Hdh^{CAG/+} ni sur les souris de type sauvage. Les inventeurs ont ensuite étudié les comportements liés à l'anxiété et à la dépression à l'aide du paradigme de novelty-suppressed feeding NSF (test de l'alimentation en environnement nouveau après privation de nourriture) qui est révélateur pour les stades anxieux et dépressifs (FIG 5C). La latence jusqu'à la prise de nourriture dans un nouvel environnement pour les souris Hdh^{CAG/+} était significativement plus longue que celle chez les souris de type sauvage non traitées. De manière intéressante, les souris Hdh^{CAG/+} traitées par ML-348 se comportaient comme des souris de type sauvage, ce qui suggère que ML-348 a un effet significatif sur les comportements anxio-dépressifs induits par la maladie de Huntington (FIG 5C).
** ML-348 chronique a des effets bénéfiques sur les déficits moteurs chez les souris Hdh* ^{*CAG140*/*+*}

Les inventeurs ont ensuite déterminé les effets de ML-348 sur le comportement moteur. L'activité locomotrice spontanée des souris a été évaluée à l'aide du test Open Field (OF). La distance ambulatoire totale parcourue était légèrement inférieure pour les souris Hdh^{CAG/+} par comparaison aux souris de type sauvage, bien que la différence ne soit pas significative à ce stade. ML-348 n'avait pas d'effet sur cette tâche (FIG 5D). Les inventeurs ont ensuite étudié la coordination motrice à l'aide du test d'Horizontal Ladder (HL). Les souris Hdh^{CAG/+} ont montré des défauts significatifs dans cette tâche, tel que le montre le taux d'erreurs élevé et la durée moyenne pour traverser l'échelle horizontale (FIG 5E). Une amélioration significative a été observée chez les souris Hdh^{CAG/+} traitées par ML-348, comme le montrent les diminutions des valeurs de score d'erreurs et de la durée pour réaliser la tâche qui sont similaires aux valeurs chez les souris de type sauvage (FIG 5E). Finalement, pour confirmer l'effet bénéfique de ML-348 sur la coordination motrice, les inventeurs ont exploré l'équilibre et la coordination motrice à l'aide du test rotarod. Une vitesse élevée en rotarod fixe ne permet pas de montrer de différence entre les souris de type sauvage et les souris Hdh ^{CAG/+} (FIG 5F). De manière intéressante, les souris Hdh^{CAG/+} présentent des altérations significatives de la coordination motrice selon le test rotarod, à la fois à des vitesses accélérées (condition 300s) et des vitesses constantes (10 et 15 rpm) (FIG 5F). Dans ces trois tests, ML-348 a amélioré significativement les performances des souris Hdh^{CAG/+} puisque les valeurs étaient similaires à celles des souris WT non traitées (FIG 5F).

Ces résultats chez les souris Hdh^{CAG/+} indiquent qu'une administration constante de ML-348 à une dose de 0,3 mg/kg pendant un mois a des effets significatifs sur la coordination motrice.
** ML-348 restaure le trafic de BDNF dans les neurones corticaux issus d'iPSC humaines provenant de patients atteints de la maladie de Huntington*

Les résultats présentés à la Figure 7 indiquent qu'un traitement par ML-348 sur des neurones corticaux d'origine humaine obtenus en culture permet de restaurer le transport axonal défectueux rencontré dans la maladie de Huntington. Ces résultats sont prometteurs quant à l'efficacité thérapeutique de ML-348 sur les maladies neurodégénératives affectant le trafic intracellulaire.

### REFERENCES BIBLIOGRAPHIQUES

- Rubinsztein, D C, et al. 1996. "Phenotypic Characterization of Individuals with 30-40 CAG Repeats in the Huntington Disease (HD) Gene Reveals HD Cases with 36 Repeats and Apparently Normal Elderly Individuals with 36-39 Repeats." American Journal of Human Genetics 59 (1): 16-22.
- Gencik, M., et al. 2002. "Chorea Huntington: A Rare Case with Childhood Onset." Neuropediatrics 33 (2): 90-92.
- Gauthier, Laurent R, et al. 2004. "And Survival of Neurons by Enhancing BDNF Vesicular Transport along Microtubules." Cell 118: 127-38.
- Dorsey, E, et al. 2013. "Natural History of Huntington Disease." JAMA Neurology 70 (12): 1520-30.
- Paulsen, Jane S. 2011. "Cognitive Impairment in Huntington Disease: Diagnosis and Treatment." Current Neurology and Neuroscience Reports 11 (5): 474.
- Papp, Kathryn V, Richard F Kaplan, and Peter J Snyder. 2011. "Biological Markers of Cognition in Prodromal Huntington's Disease: A Review." Brain and Cognition 77(2):280-91.
- Young, Fiona B, et al. 2011. "Putting Proteins in Their Place: Palmitoylation in Huntington Disease and Other Neuropsychiatric Diseases." Progress in Neurobiology 97 (2). Elsevier Ltd: 220-38.
- Tse, David, et al. 2015. "ABHD17 Proteins Are Novel Protein Depalmitoylases That Regulate N-Ras Palmitate Turnover and Subcellular Localization . Centre for Molecular Medicine & Therapeutics , Department of Medical Genetics , University of British Columbia , 950 West 28 Th Avenue." eLife 10 (December)
- Tomatis, Vanesa M, et al. 2010. "Acyl-Protein Thioesterase 2 Catalizes the Deacylation of Peripheral Membrane-Associated GAP-43." PLOS ONE 5 (11).
- Zeidman, Ruth, et al. 2009. "Protein Acyl Thioesterases." Molecular Membrane Biology 26 (1-2). Taylor & Francis: 32-41.
- Hirano, Tohko, et al. 2009. "Thioesterase Activity and
- Subcellular Localization of Acylprotein Thioesterase 1/lysophospholipase 1." Biochimica et Biophysica Acta (BBA) - Molecular and Cell Biology of Lipids 1791 (8):797-805.
- Yanai, Anat, et al. 2006. "Palmitoylation of Huntingtin by HIP14 Is Essential for Its Trafficking and Function." Nature Neuroscience 9 (6). Nature Publishing Group: 824-31.
- Young, Fiona B, et al. 2012. "Low Levels of Human HIP14 Are Sufficient to Rescue Neuropathological, Behavioural, and Enzymatic Defects Due to Loss of Murine HIP14 in Hip14-/- Mice." PLoS ONE 7 (5): e36315.
- Adibekian, Alexander, et al. 2012. "Confirming Target Engagement for Reversible Inhibitors in Vivo by Kinetically Tuned Activity-Based Probes." Journal of the American Chemical Society 134 (25): 10345-48.
- Vujic et al, 2015. « Acyl protein thioesterase 1 and 2 (APT-1, APT-2) inhibitors palmostatin B, ML348 and ML349 have different effects on NRAS mutant melanoma cells » Oncotarget, 2016 Feb 9; 7(6): 7297-7306
- Boncompain, Gaelle, et al. 2012. "Synchronization of Secretory Protein Traffic in Populations of Cells." Nature Methods 9 (5) : 493-98.

## Revendications

1. - Inhibiteur de l'acyl-thioestérase APT1, ML-348, de formule A : 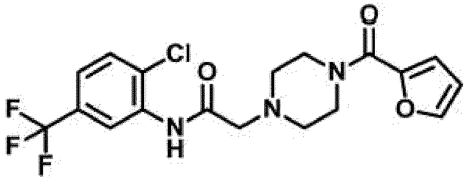 pour son utilisation dans le traitement et/ou la prévention d'une maladie neurodégénérative ou d'un trouble neurodégénératif.

2. - Inhibiteur de l'acyl-thioesthérase APT1 pour son utilisation selon la revendication 1, dans lequel la maladie neurodégénérative ou le trouble neurodégénératif est la maladie de Huntington.

3. - Inhibiteur de l'acyl-thioesthérase APT1 pour son utilisation selon larevendication 1, dans lequel la maladie neurodégénérative ou le trouble neurodégénératif est le syndrome de Rett.

4. - Inhibiteur de l'acyl-thioesthérase APT1 pour son utilisation selon l'une des revendications 1 à 3, **caractérisé par le fait que** l'inhibiteur est destiné à être administré au sujet selon une quantité thérapeutiquement efficace.

5. - Composition pharmaceutique pour son utilisation selon l'une des revendications 1 à 3 comprenant :
- au moins l'inhibiteur de l'acyl-thioestérase APT1 selon la revendication 1; et
- au moins un excipient pharmaceutiquement acceptable.

6. - Composition pour son utilisation selon la revendication 5, **caractérisée par le fait que** la composition est destinée à être administrée au sujet selon une quantité thérapeutiquement efficace.

7. - Inhibiteur de l'acyl-thioestérase APT1 selon la revendication 1 pour son utilisation dans le traitement et/ou la prévention d'une maladie ou d'un trouble lié(e) à un trafic intracellulaire altéré entre le réticulum endoplasmique et l'appareil de Golgi et entre celui-ci et la membrane plasmique, la maladie ou le trouble étant l'un(e) choisi(e) parmi la maladie de Huntington, la maladie de Parkinson, la Sclérose Latérale Amyotrophique ou maladie de Charcot (SLA), le syndrome de Rett, la maladie de Charcot-Marie-Tooth, la neuropathie axonale sensitivomotrice, le syndrome de Perry, la maladie d'Alzheimer précoce autosomique dominante (AD3), le syndrome de paralysie supranucléaire progressive (PSP), la paraplégie spastique autosomique.

8. - Composition pharmaceutique selon l'une des revendications 5 ou 6 pour son utilisation dans le traitement et/ou la prévention d'une maladie ou d'un trouble lié(e) à un trafic intracellulaire altéré entre le réticulum endoplasmique et l'appareil de Golgi et entre celui-ci et la membrane plasmique, la maladie ou le trouble étant l'un(e) choisi(e) parmi la maladie de Huntington, la maladie de Parkinson, la Sclérose Latérale Amyotrophique ou maladie de Charcot (SLA), le syndrome de Rett, la maladie de Charcot-Marie-Tooth, la neuropathie axonale sensitivomotrice, le syndrome de Perry, la maladie d'Alzheimer précoce autosomique dominante (AD3), le syndrome de paralysie supranucléaire progressive (PSP), la paraplégie spastique autosomique.

## Patentansprüche

1. - APT1-Acylthioesterase-Inhibitor ML-348 von Formel A: 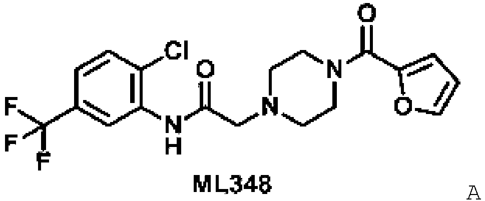 zur Verwendung bei der Behandlung und/oder Prävention einer neurodegenerativen Erkrankung oder einer neurodegenerativen Störung.

2. - APT1-Acylthioesterase-Inhibitor zur Verwendung nach Anspruch 1, wobei die neurodegenerative Erkrankung oder die neurodegenerative Störung die Huntington-Krankheit ist.

3. - APT1-Acylthioesterase-Inhibitor zur Verwendung nach Anspruch 1, wobei die neurodegenerative Erkrankung oder die neurodegenerative Störung das Rett-Syndrom ist.

4. - APT1-Acylthioesterase-Inhibitor zur Verwendung nach einem der Ansprüche 1 bis 3, **dadurch gekennzeichnet, dass** der Inhibitor dazu bestimmt ist, dem Subjekt in einer therapeutisch wirksamen Menge verabreicht zu werden.

5. - Zusammensetzung zur Verwendung nach Anspruch 1 bis 3, umfassend:
- mindestens den APT1-Acylthioesterase-Inhibitor nach Anspruch 1; und
- mindestens einen pharmazeutisch annehmbarer Hilfsstoff.

6. - Zusammensetzung zur Verwendung nach Anspruch 5, **dadurch gekennzeichnet, dass** die Zusammensetzung dazu bestimmt ist, dem Subjekt in einer therapeutisch wirksamen Menge verabreicht zu werden.

7. - APT1-Acylthioesterase-Inhibitor nach Anspruch 1 zur Verwendung bei der Behandlung und/oder Prävention einer Erkrankung oder Störung, die mit einem gestörten intrazellulären Transport zwischen dem endoplasmatischen Retikulum und dem Golgi-Apparat und zwischen diesem und der Plasmamembran verbunden ist, wobei die Erkrankung oder Störung ausgewählt ist aus der Gruppe, bestehend aus Huntington-Krankheit, Parkinson-Krankheit, Amyotrophe Lateralsklerose oder Charcot-Krankheit (ALS), Rett-Syndrom, Morbus Charcot-Marie-Tooth, sensomotorischer axonaler Neuropathie, Perry-Syndrom, autosomal-dominanter früh einsetzender Alzheimer-Krankheit (AD3), progressivem supranukleärem Paralyse-Syndrom (PSP), autosomalspastischer Paraplegie.

8. - Pharmazeutische Zusammensetzung nach einem der Ansprüche 5 oder 6 zur Verwendung bei der Behandlung und/oder Prävention einer Erkrankung oder Störung, die mit einem gestörten intrazellulären Transport zwischen dem endoplasmatischen Retikulum und dem Golgi-Apparat und zwischen diesem und der Plasmamembran verbunden ist, wobei die Erkrankung oder Störung ausgewählt ist aus der Gruppe, bestehend aus Huntington-Krankheit, Parkinson-Krankheit, Amyotrophe Lateralsklerose oder Charcot-Krankheit (ALS), Rett-Syndrom, Morbus Charcot-Marie-Tooth, sensomotorischer axonaler Neuropathie, Perry-Syndrom, autosomal-dominanter früh einsetzender Alzheimer-Krankheit (AD3), progressivem supranukleärem Paralyse-Syndrom (PSP), autosomalspastischer Paraplegie.

## Claims

1. - Acyl-protein thioesterase APT1 inhibitor, ML-348, of formula A : 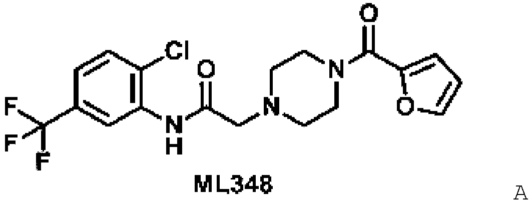 for use in the treatment and/or prevention of a neurodegenerative disease or a neurodegenerative disorder.

2. - The acyl-protein thioesterase APT1 inhibitor for use according to claim 1, wherein the neurodegenerative disease or neurodegenerative disorder is Huntington's disease.

3. - The acyl-protein thioesterase APT1 inhibitor for use according to claim 1, wherein the neurodegenerative disease or neurodegenerative disorder is Rett's syndrome.

4. - The acyl-protein thioesterase APT1 inhibitor for use according to any one of claims 1 to 3, wherein the inhibitor is for administration to the subjet in a therapeutically effective amount.

5. - A pharmaceutical composition for use according to any one of claims 1 to 3 comprising:
- at least the acyl-protein thioesterase APT1 inhibitor according to claim 1 ; and
- at least one pharmaceutically acceptable excipient.

6. - Composition for use according to claim 5, wherein the composition is for administration to the subject in a therapeutically effective amount.

7. - The acyl-protein thioesterase APT1 inhibitor according to claim 1 for use in the treatment and/or prevention of a disease or disorder related to impaired intracellular trafficking between the endoplasmic reticulum and the Golgi apparatus and between the latter and the plasma membrane, the disease or disorder being one selected from Huntington's disease, Parkinson's disease, Amyotrophic Lateral Sclerosis or Charcot's disease (ALS), Rett's syndrome, Charcot-Marie-Tooth disease, motor-sensory axonal neuropathy, Perry's syndrome, early-onset autosomal dominant Alzheimer's disease (AD3), progressive supranuclear palsy syndrome (PSP), autosomal spastic paraplegia.

8. - The pharmaceutical composition according to claim 5 or 6 for use in the treatment and/or prevention of a disease or disorder related to impaired intracellular trafficking between the endoplasmic reticulum and the Golgi apparatus and between the latter and the plasma membrane, the disease or disorder being one selected from Huntington's disease, Parkinson's disease, Amyotrophic Lateral Sclerosis or Charcot's disease (ALS), Rett's syndrome, Charcot-Marie-Tooth disease, motor-sensory axonal neuropathy, Perry's syndrome, early-onset autosomal dominant Alzheimer's disease (AD3), progressive supranuclear palsy syndrome (PSP), autosomal spastic paraplegia.
